(19) Europäisches Patentamt
European Patent Office
Office européen des brevets

(11) Veröffentlichungsnummer: **0 362 632 A2**

(12) # EUROPÄISCHE PATENTANMELDUNG

(21) Anmeldenummer: 89117494.8

(51) Int. Cl.5: C07D 211/90 , C07D 401/12 , A61K 31/44

(22) Anmeldetag: 21.09.89

(30) Priorität: 05.10.88 DE 3833892

(43) Veröffentlichungstag der Anmeldung: 11.04.90 Patentblatt 90/15

(84) Benannte Vertragsstaaten: AT BE CH DE ES FR GB GR IT LI LU NL SE

(71) Anmelder: BAYER AG
D-5090 Leverkusen 1 Bayerwerk(DE)

(72) Erfinder: Stoltefuss, D. I.
Parkstrasse 20
D-5657 Haan(DE)
Erfinder: Schwenner, Eckhard, Dr.
Paul-Ehrlich-Strasse 29
D-5600 Wuppertal 1(DE)
Erfinder: Gross, Rainer, Prof. Dr.
Platzhoffstrasse 23
D-5600 Wuppertal(DE)
Erfinder: Hebisch, Siegbert, Dr.
Richard-Seel-Weg 11
D-5600 Wuppertal(DE)
Erfinder: Schramm, Matthias, Dr.
Paffrather Strasse 38
D-5000 Köln 80(DE)
Erfinder: Bechem, Martin, Dr.
Bergerheide 4
D-5600 Wuppertal 1(DE)
Erfinder: Hirth, Claudia, Dr.
Claudiusweg 9
D-5600 Wuppertal 1(DE)
Erfinder: Stasch, Johannes-Peter, Dr.
Schneewittchenweg 37
D-5600 Wuppertal 1(DE)

(54) **Basische 4-Aryl-DHP-amide, Verfahren zu ihrer Herstellung und ihre Verwendung in Arzneimitteln.**

(57) Die vorliegende Erfindung betrifft Dihydropyridinamide der allgemeinen Formel (I)

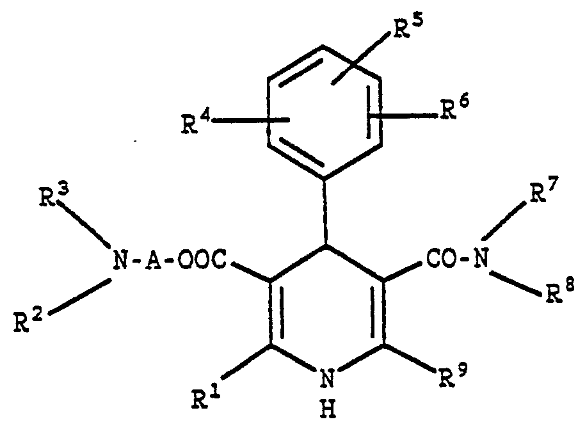

(I)

in welcher $R^1$ bis $R^9$ und A die in der Beschreibung angegebene Bedeutung haben, Verfahren zu ihrer Herstellung sowie ihre Verwendung in Arzneimitteln, insbesondere als kreislaufbeeinflussende Arzneimittel.

EP 0 362 632 A2

Xerox Copy Centre

## Basische 4-Aryl-DHP-amide, Verfahren zu ihrer Herstellung und ihre Verwendung in Arzneimitteln

Die vorliegende Erfindung betrifft Dihydropyridinamide, Verfahren zu ihrer Herstellung sowie ihre Verwendung in Arzneimitteln, insbesondere als kreislaufbeeinflussende Arzneimittel.

Es ist bekannt, daß man 1,4-Dihydro-2,6-dimethyl-4-phenyl-pyridin-3,5-dicarbonsäurediethylester erhält, wenn man Benzylidenacetessigsäureethylester mit ß-Aminocrotonsäureethylester oder Acetessigsäureethylester und Ammoniak umsetzt [E. Knoevenagel, Ber. Dtsch. Chem. Ges. 31, 743 (1898)].

Weiterhin ist bekannt, daß bestimmte 1,4-Dihydropyridine interessante pharmakologische Eigenschaften aufweisen [F. Bossert, W. Vater, Naturwissenschaften 58, 578 (1971)]. Aus EP-A 220 653 ist bekannt, daß man 3-Aminocarbonyl-1,4-dihydropyridin-5-carbonsäurederivate erhält, wenn man Alkyl-o(oder m-)nitrobenzylidenacetoacetate mit 3-Aminocrotonamiden in einer Cyclisierungsreaktion umsetzt.

Die vorliegende Erfindung betrifft neue Dihydropyridinamide der allgemeinen Formel (I)

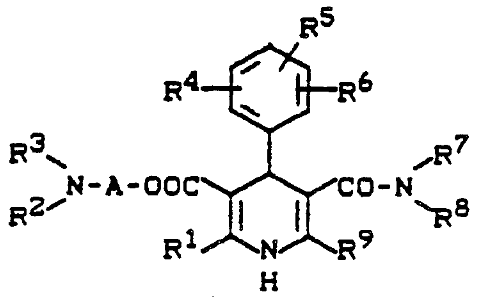

(I)

in welcher

$R^1$ und $R^9$ gleich oder verschieden sind und

- für geradkettiges, verzweigtes oder cyclisches Alkyl mit bis zu 6 Kohlenstoffatomen stehen, das gegebenenfalls durch Hydroxy, Cyano, Phenyl oder Halogen substituiert ist, oder
- für Cyano oder Phenyl stehen,

$R^2$ und $R^3$ gleich oder verschieden sind und für Wasserstoff, für geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, durch Phenyl, welches gegebenenfalls durch Nitro, Phenyl, Cyano, Trifluormethyl, Trifluormethoxy, Alkyl mit bis zu 4 Kohlenstoffatomen, Halogen oder Alkoxy mit bis zu 4 Kohlenstoffatomen substituiert ist, für Cycloalkyl mit 3 bis 8 Kohlenstoffatomen oder

- für Aryl mit 6 bis 12 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe, Acetylamino oder Benzoylamino substituiert sein kann, oder

$R^2$ und $R^3$ zusammen einen 5- bis 7-gliedrigen gesättigten oder ungesättigten heterocyclischen Ring bilden, der als Heteroatom ein Sauerstoffatom, ein Schwefelatom oder ein zusätzliches Stickstoffatom enthalten kann, welches gegebenenfalls durch eine Gruppe $R^{13}$ substituiert sein kann, die für Wasserstoff, eine geradkettige oder verzweigte, gesättigte oder ungesättigte Alkylgruppe mit bis zu 10 Kohlenstoffatomen steht, die gegebenenfalls durch Phenyl, welches durch Halogen, Alkyl mit bis zu 4 C-Atomen, Alkoxy mit bis zu 4 C-Atomen, Nitro und Halogenalkyl mit bis zu 2 C-Atomen substituiert sein kann oder für Phenyl steht, das gegebenenfalls durch Halogen, Cyano, Nitro, Alkyl mit bis zu 2 C-Atomen, Alkoxy mit bis zu 2 C-Atomen oder Halogenalkyl mit bis zu 2 C-Atomen substituiert sein kann,

$R^4$ und $R^5$ gleich oder verschieden sind und

- für Wasserstoff, Halogen, Alkyl mit bis zu 6 Kohlenstoffatomen, Alkoxy mit bis zu 6 Kohlenstoffatomen, Alkylthio mit bis zu 4 Kohlenstoffatomen, Cyano, Nitro, Dialkylamino mit jeweils bis zu 4 Kohlenstoffatomen je Alkylgruppe, Trifluormethyl, Trifluormethoxy, Difluormethoxy oder Trifluormethylthio stehen,

$R^6$ - für eine Gruppe der Formel $-O-(CH_2)n-R^{10}$, $-S-(CH_2)_n-R^{10}$, $-O-SO_2-(CH_2)_nR^{10}$, $-O-CO-(CH_2)_n-R^{10}$ steht, worin

n - 0 bis 4 bedeutet,

und

$R^{10}$- Cyclohexyl oder Aryl mit 6 bis 10 Kohlenstoffatomen bedeutet, das bis zu 4-fach gleich oder verschieden durch Halogen, Cyano, Nitro, Trifluormethyl, Trifluormethoxy, Difluormethoxy, Trifluormethylthio, Alkyl mit bis zu 6 Kohlen stoffatomen, Alkoxy mit bis zu 6 Kohlenstoffatomen, Alkylthio mit bis zu 4 Kohlenstoffatomen, Amino, Alkylamino mit bis zu 6 Kohlenstoffatomen, Dialkylamino mit jeweils bis zu 6 Kohlenstoffatomen je Alkylgruppe oder Acetylamino substituiert sein kann oder

- einen 5- bis 7-gliedrigen gesättigten oder ungesättigten heterocyclischen Ring bedeutet, der als Heteroatome ein Sauerstoffatom, ein Schwefelatom oder zwei Stickstoffatome enthalten kann,

und

$R^7$ und $R^8$ gleich oder verschieden sind und jeweils

- für Wasserstoff,
- für Cycloalkyl mit 3 bis 8 Kohlenstoffatomen oder
- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^{11}R^{12}$ substituiert sind,

worin

$R^{11}$ und $R^{12}$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 10 Kohlenstoffatomen, Acetyl, Benzoyl, Alkylsulfonyl mit bis zu 6 Kohlenstoffatomen oder Phenylsulfonyl bedeuten,

oder $R^7$ und $R^8$ jeweils

- für Aryl mit 6 bis 10 Kohlenstoffatomen steht, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 4 Kohlenstoffatomen, Dialkylamino mit bis zu 4 Kohlenstoffatomen je Alkylgruppe, Acetylamino oder Benzoylamino substituiert ist, oder
- für einen 5- bis 7-gliedrigen gesättigten oder ungesättigten heterocyclischen Ring stehen, der als zusätzliches Heteroatom ein Sauerstoffatom, ein Schwefelatom oder eine NH- oder N-Alkyl-Gruppe (1-4 C-Atome) enthalten kann, und

A für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^{14}$ unterbrochen sein kann, in der

$R^{14}$ Wasserstoff, Alkyl bis zu 4 Kohlenstoffatomen, Benzyl oder Phenethyl bedeuten kann,

und/oder

der substituiert sein kann durch Halogen, Hydroxy, Acetoxy, Carboxy, Alkoxycarbonyl mit bis zu 8 Kohlenstoffatomen oder Phenyl, das gegebenenfalls substituiert sein kann durch Halogen, Alkyl mit bis zu 2 Kohlenstoffatomen, Alkoxy mit bis zu 2 Kohlenstoffatomen, Halogenmethyl, Halogenmethoxy, Hydroxy oder Cyano,

und deren physiologisch unbedenklichen Salze, in Form ihrer Diastereomerengemische, Racemformen und Antipoden.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Bevorzugt sind Verbindungen der allgemeinen Formel (I),

in welcher

$R^1$ und $R^9$ gleich oder verschieden sind und jeweils
- für geradkettiges oder verzweigtes Alkyl mit bis zu 4 Kohlenstoffatomen stehen, das gegebenenfalls durch Hydroxy oder Phenyl substituiert ist,
$R^2$ und $R^3$ gleich oder verschieden sind und für Wasserstoff, für geradkettiges oder verzweigtes Alkyl, Alkenyl oder Alkinyl mit jeweils bis zu 8 Kohlenstoffatomen stehen, das substituiert sein kann, durch Halogen, Hydroxy, Alkoxy mit bis zu 4 Kohlenstoffatomen, Cyano, Trifluormethyl, Alkylthio mit bis zu 4 Kohlenstoffatomen, Alkylcarbonyl mit bis zu 4 Kohlenstoffatomen im Alkylrest, Carboxy oder Alkoxycarbonyl mit bis zu 8 Kohlenstoffatomen, durch Phenyl, welches gegebenenfalls durch Nitro, Phenyl, Trifluormethyl, Trifluormethoxy, Alkyl mit bis zu 2 Kohlenstoffatomen, Halogen oder Alkoxy mit bis zu 2 Kohlenstoffatomen substituiert ist, für Cycloalkyl mit 3 bis 8 Kohlenstoffatomen oder
- für Aryl mit 6 bis 10 Kohlenstoffatomen stehen, das bis zu 2-fach gleich oder verschieden durch Nitro, Cyano, Halogen, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Difluormethoxy, Trifluormethylthio, Amino, Alkylamino mit bis zu 2 Kohlenstoffatomen, Dialkylamino mit jeweils bis zu 2 Kohlenstoffatomen je Alkylgruppe, Acetylamino oder Benzoylamino substituiert sein kann,
oder
$R^2$ und $R^3$ einen 5- bis 7-gliedrigen gesättigten oder ungesättigten heterocyclischen Ring bilden, der als Heteroatom ein Sauerstoffatom, ein Schwefelatom oder ein zusätzliches Stickstoffatom enthalten kann, welches gegebenfalls durch eine Gruppe $R^{13}$ substituiert sein kann, die für Wasserstoff, eine geradkettige oder verzweigte, gesättigte oder ungesättigte Alkylgruppe mit bis zu 10 Kohlenstoffatomen steht, die gegebenenfalls durch Phenyl, welches durch Halogen, Alkyl mit bis zu 4 C-Atomen, Alkoxy mit bis zu 4 C-Atomen, Nitro und Halogenalkyl mit bis zu 2 C-Atomen substituiert sein kann oder für Phenyl steht, das gegebenenfalls durch Halogen, Cyano, Nitro, Alkyl mit bis zu 2 C-Atomen, Alkoxy mit bis zu 2 C-Atomen oder Halogenalkyl mit bis zu 2 C-Atomen substituiert sein kann,
$R^4$ und $R^5$ gleich oder verschieden sind und
- für Wasserstoff, Fluor, Chlor, Brom, Alkyl mit bis zu 4 Kohlenstoffatomen, Alkoxy mit bis zu 4 Kohlenstoffatomen, Methylthio, Cyano, Nitro, Trifluormethyl oder Trifluormethoxy stehen,
$R^6$ - für eine Gruppe der Formel $-O-(CH_2)n-R^{10}$, $-S-(CH_2)_n-R^{10}$, $-O-SO_2-(CH_2)_n-R^{10}$ oder $-O-CO-(CH_2)_n-R^{10}$ steht,
worin
n - 0 bis 3 bedeutet,
und
$R^{10}$- Cyclohexyl oder Phenyl bedeutet, das bis zu 3-fach gleich oder verschieden substituiert sein kann durch Fluor, Chlor, Brom, Cyano, Nitro, Trifluormethyl, Trifluormethoxy, Alkyl mit bis zu 4 Kohlenstoffatomen, Alkoxy mit bis zu 4 Kohlenstoffatomen, Methylthio, Amino, Alkylamino mit bis zu 4 Kohlenstoffatomen, Dialkylamino mit jeweils bis zu 4 Kohlenstoffatomen je Alkylgruppe, Acetylamino, oder
- Pyridyl, Thienyl, Furyl, Pyrimidyl oder Pyrazinyl bedeutet,
und
$R^7$ und $R^8$ gleich oder verschieden sind und jeweils
- für Wasserstoff, Cycloalklyl mit 3 bis 7 Kohlenstoffatomen stehen oder
- für geradkettiges oder verzweigtes Alkyl oder Alkenyl mit bis zu 14 Kohlenstoffatomen stehen, die substituiert sein können durch Fluor, Chlor, Brom, Hydroxy, Alkoxy mit bis zu 6 Kohlenstoffatomen, Alkylthio mit bis zu 6 Kohlenstoffatomen, Alkylcarbonyl mit bis zu 6 Kohlenstoffatomen im Alkylrest, Carboxy, Alkoxycarbonyl mit bis zu 6 Kohlenstoffatomen, durch gegebenenfalls durch Nitro, Trifluormethyl, Methyl oder Methoxy substituierte Phenyl- oder Phenoxy-Rest oder wobei die Reste Alkyl und Alkenyl gegebenenfalls substituiert sind durch Cyano und/oder durch eine Gruppe der Formel $-NR^{11}R^{12}$,
worin
$R^{11}$ und $R^{12}$ gleich oder verschieden sind und jeweils Wasserstoff, Alkyl mit bis zu 6 Kohlenstoffatomen, Benzyl, Phenethyl, Phenyl, Acetyl, Benzoyl, Alkylsulfonyl mit bis zu 4 Kohlenstoffatomen oder Phenylsulfonyl bedeuten,
oder
- für Phenyl oder Naphthyl stehen, die bis zu 3-fach gleich oder verschieden substituiert sein können durch Nitro, Fluor, Chlor, Brom, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino, oder
- für Pyrrolidino, Pyridino, Morpholino oder für Piperazino, $N-C_1-C_4$-Alkylpiperazino, $N-C_7-C_9$-Aralkyl- oder N-Phenyl-piperazino stehen,

und
A für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe N-$R^{14}$ unterbrochen sein kann, in der
$R^{14}$ Wasserstoff, Alkyl bis zu 2 Kohlenstoffatomen, Benzyl oder Phenethyl bedeuten kann,
und/oder
der substituiert sein kann durch Halogen, Hydroxy, Acetoxy, Carboxy, Alkoxycarbonyl mit bis zu 4 Kohlenstoffatomen oder durch Phenyl, das gegebenenfalls substituiert sein kann durch Halogen, Alkyl mit bis zu 2 Kohlenstoffatomen, Alkoxy mit bis zu 2 Kohlenstoffatomen, Trifluormethyl oder Trifluormethoxy sowie deren physiologisch unbedenklichen Salze.

Besonders bevorzugt sind Verbindungen der allgemeinen Formel (I), in welcher
$R^1$ und $R^9$ gleich oder verschieden sind und für Methyl, Ethyl oder Benzyl stehen,
$R^2$ und $R^3$ gleich oder verschieden sind und für Wasserstoff, für geradkettiges oder verzweigtes Alkyl oder Alkenyl mit bis zu 6 Kohlenstoffatomen stehen, das substituiert sein kann durch Hydroxy, Carboxy, Alkoxycarbonyl mit bis zu 4 C-Atomen im Alkylrest, durch Phenyl, welches gegebenenfalls durch Nitro, Halogen, Trifluormethyl, Trifluormethoxy, Methyl oder Methoxy substituiert ist, für Cycloalkyl mit 3 bis 6 Kohlenstoffatomen oder
- für Phenyl stehen, das bis zu 2-fach gleich oder verschieden durch Nitro, Halogen, Alkyl mit bis zu 2 Kohlenstoffatomen, Alkoxy mit bis zu 2 Kohlenstoffatomen, Alkylthio mit bis zu 2 Kohlenstoffatomen, Trifluormethyl, Trifluormethoxy, Difluormethoxy, Trifluormethylthio, Amino, Alkylamino mit bis zu 2 Kohlenstoffatomen oder Dialkylamino mit jeweils bis zu 2 Kohlenstoffatomen je Alkylgruppe substituiert sein kann,
oder
- für einen 5- bis 7-gliedrigen gesättigten oder ungesättigten heterocyclischen Ring stehen, der als Heteroatom ein Sauerstoffatom, ein Schwefelatom oder ein zusätzliches Stickstoffatom enthalten kann, welches gegebenenfalls durch eine Gruppe $R^{13}$ substituiert sein kann, die Wasserstoff, Alkyl mit bis zu 4 C-Atomen, Benzyl, Phenethyl oder gegebenenfalls durch Halogen, Methyl oder Methoxy substituiertes Phenyl bedeutet,
$R^4$ und $R^5$ gleich oder verschieden sein können und jeweils für Wasserstoff, Fluor, Chlor, Methyl, Ethyl, Methoxy, Ethoxy, Nitro oder Trifluormethyl stehen,
$R^6$ für eine Gruppe der Formel $-O-(CH_2)_nR^{10}$, $-S-(CH_2)_nR^{10}$, $-O-SO_2-(CH_2)_n-R^{10}$ oder $-O-CO-(CH_2)_n-R^{10}$ steht,
worin
n 0 bis 2 bedeutet und
$R^{10}$- Cyclohexyl oder Phenyl bedeutet, das bis zu 2-fach gleich oder verschieden substituiert sein kann durch Fluor, Chlor, Nitro, Trifluormethyl, Methyl, Methoxy, Amino, Methylamino, Dimethylamino, Ethylamino, Diethylamino oder Acetylamino, oder
- eine $\alpha$-, $\beta$- oder eine $\gamma$-Pyridylgruppe bedeutet,
$R^7$ - für Wasserstoff oder Alkyl mit bis zu 4 Kohlenstoffatomen steht
und
$R^8$ - für Wasserstoff, Cyclopropyl, Cyclopentyl, Cyclohexyl steht, oder
- für geradkettiges oder verzweigtes Alkyl oder Alkenyl mit bis zu 10 Kohlenstoffatomen steht, das substituiert sein kann durch Fluor, Chlor, Hydroxy, Alkoxy mit bis zu 4 Kohlenstoffatomen, Alkylthio mit bis zu 4 Kohlenstoffatomen, Alkylcarbonyl mit bis zu 4 Kohlenstoffatomen im Alkylrest, Carboxy, Alkoxycarbonyl mit bis zu 4 Kohlenstoffatomen, Phenoxy, Phenyl oder die obengenannten Reste Alkyl und Alkenyl gegebenenfalls substituiert sind durch eine Gruppe der Formel $-NR^{11}R^{12}$,
worin
$R^{11}$ und $R^{12}$ gleich oder verschieden sind und Wasserstoff, Alkyl mit bis zu 4 Kohlenstoffatomen, Benzyl, Phenyl oder Acetyl bedeuten,
oder $R^8$
- für Phenyl steht, das bis zu 2-fach gleich oder verschieden substituiert sein kann durch Nitro, Fluor, Chlor, Methyl, Methoxy, Trifluormethyl, Trifluormethoxy, Amino, Alkylamino mit bis zu 2 Kohlenstoffatomen oder Dialkylamino mit jeweils bis zu 2 Kohlenstoffatomen je Alkylgruppe steht
und
A für einen geradkettigen, verzeigten oder cyclischen, gesättigten oder ungesättigten Kohlenwasserstoffrest mit bis zu 8 Kohlenstoffatomen steht, der gegebenenfalls durch ein Sauerstoff-oder Schwefelatom oder eine Gruppe N-$R^{14}$ unterbrochen ist, in der
$R^{14}$ Wasserstoff, Alkyl mit bis zu 2 Kohlenstoffatomen, Benzyl oder Phenethyl bedeuten kann,
und/oder substituiert sein kann durch Hydroxy, Carboxy, Alkoxycarbonyl mit bis zu 4 Kohlenstoffatomen

oder durch Phenyl, das gegebenenfalls substituiert sein kann durch Halogen, Methyl, Methoxy, Trifluormethyl oder Trifluormethoxy
sowie deren physiologisch unbedenklichen Salze.

Ganz besonders bevorzugt sind Verbindungen der allgemeinen Formel (I), in welcher
$R^1$ und $R^9$ jeweils für Methyl stehen
$R^2$ und $R^3$ gleich oder verschieden sind und für geradkettiges oder verzweigtes Alkyl mit bis zu 3 Kohlenstoffatomen stehen, das gegebenenfalls durch Phenyl substituiert ist, das seinerseits durch Methyl substituiert sein kann,
oder
$R^2$ und $R^3$ gemeinsam für Morpholino stehen
$R^4$ und $R^5$ jeweils für Wasserstoff stheen
$R^6$ für eine Gruppe der Formel
$-O-(CH_2)_n-R^{10}$,$-S-(CH_2)_n-R^{10}$,$-O-SO_2-(CH_2)_n-R^{10}$ oder $-O-CO-(CH_2)_n-R^{10}$
worin
n die Zahl 0, 1 oder 2 bedeutet und
$R^{10}$ Cyclohexyl oder Phenyl bedeutet, das gegebenenfalls durch Methyl substituiert ist
$R^7$ für Wasserstoff oder Alkyl mit bis zu 4 C-Atomen sthet
$R^8$ für Wasserstoff, Cyclopropyl, Cyclopentyl oder für geradkettiges oder verzweigtes Alkyl mit bis zu 5 Kohlenstoffatomen steht, das gegebenenfalls durch Hydroxy substituiert ist, und
A für die $CH_2-CH_2$-Gruppe steht,
und deren physiologisch unbedenklichen Salze.

Die erfindungsgemäßen Verbindungen der allgemeinen Formel (I), in welcher
$R^1$ bis $R^9$ und A die oben angegebene Bedeutung haben,
erhält man, indem man
[A] Aldehyde der allgemeinen Formel (II)

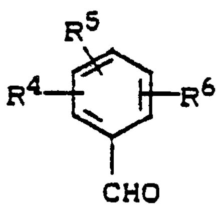

(II)

in welcher
$R^4$, $R^5$ und $R^6$ die oben angegebene Bedeutung haben
mit
$\beta$-Ketocarbonsäureestern der allgemeinen Formel (III)

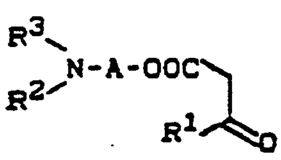

(III)

in welcher
$R^1$, $R^2$, $R^3$ und A die oben angegebene Bedeutung haben
gegebenenfalls nach Isolierung der hieraus entstehenden Ylidenverbindungen der allgemeinen Formel (IV)

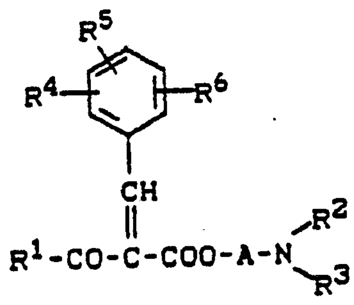 (IV)

in welcher
$R^1$, $R^2$, $R^3$, $R^4$, $R^5$, $R^6$ und A die oben angegebene Bedeutung haben,
mit
$\beta$-Ketocarbonsäureamiden der allgemeinen Formel (V)

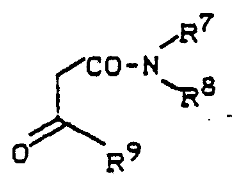 (V)

in welcher
$R^7$, $R^8$, und $R^9$ die oben angegebene Bedeutung haben
und Ammoniak bzw. direkt mit den hieraus hergestellten $\beta$-Aminocrotonsäureamiden der allgemeinen Formel (VI)

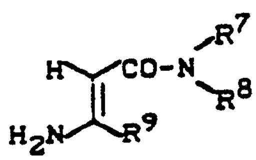 (VI)

in welcher
$R^7$, $R^8$ und $R^9$ die oben angegebene Bedeutung haben,
umsetzt,
oder indem man
[B] Aldehyde der allgemeinen Formel (II) und $\beta$-Ketocarbonsäureamiden der allgemeinen Formel (V) bzw. deren Ylidenverbindungen der allgemeinen Formel (VII)

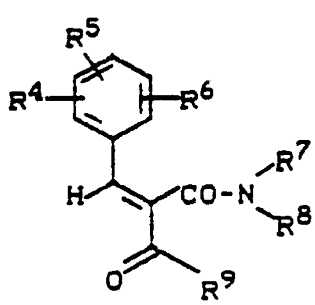 (VII)

in welcher
$R^4$, $R^5$, $R^6$, $R^7$, $R^8$ und $R^9$ die oben angegebene Bedeutung haben,
mit
$\beta$-Ketocarbonsäureestern der allgemeinen Formel (III) und Ammoniak bzw. direkt mit den hieraus hergestellten Aminocrotonsäureestern der allgemeinen Formel (VIII)

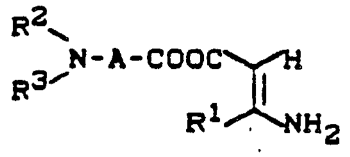

(VIII)

in welcher
$R^1$, $R^2$, $R^3$ und A die oben angegebene Bedeutung haben,
umsetzt
oder indem man
[C] Dihydropyridinmonocarbonsäuren der allgemeinen Formel (IXa) oder (IXb)

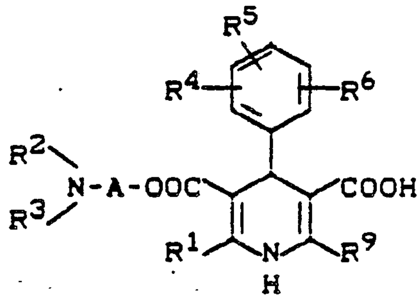

oder

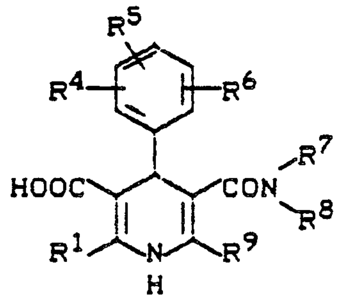

(IXa) (IXb)

in welchen
$R^1$, $R^2$, $R^3$, $R^4$, $R^5$, $R^6$, $R^7$, $R^8$, $R^9$ und A die oben angegebene Bedeutung haben,
gegebenenfalls über reaktive Säurederivate mit Aminen der allgemeinen Formel (Xa)

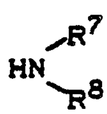

(Xa)

in welcher
$R^7$ und $R^8$ die oben angegebene Bedeutung haben,
bzw. mit Verbindungen der Formel (Xb)

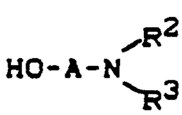

(Xb)

in welcher
$R^2$, $R^3$ und A die oben angegebene Bedeutung haben,
umsetzt, wobei selbstverständlich jeweils nur (IXa) mit (Xa) und (IXb) mit (Xb) umgesetzt wird.

Enantiomerenreine Verbindungen der Formel (I) erhält man beispielsweise, indem man Diastereomerengemische der Formeln (XIa*) bzw. (XIb*) ,

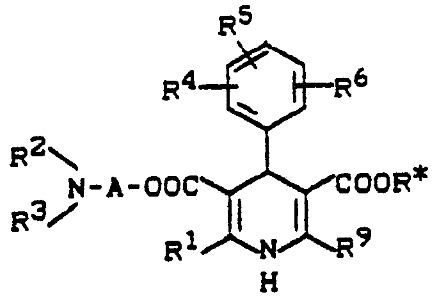

und

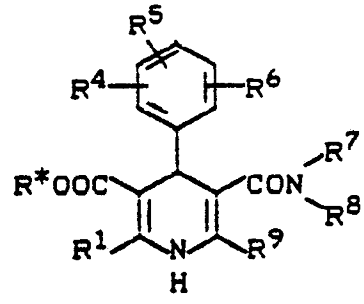

(XIa*) (XIb*)

in welcher
$R^1$, $R^2$, $R^3$, $R^4$, $R^5$, $R^6$, $R^7$, $R^8$, $R^9$ und A die oben angegebene Bedeutung haben und
$R^*$ einen optisch aktiven Esterrest darstellt, durch Kristallisation, Chromatographie oder Craig-Verteilung in die einzelnen Diastereomeren trennt,
gegebenenfalls den optisch aktiven Esterrest abspaltet und anschließend die enantiomerenreinen Carbonsäuren der Formeln (IXa*) und (IXb*)

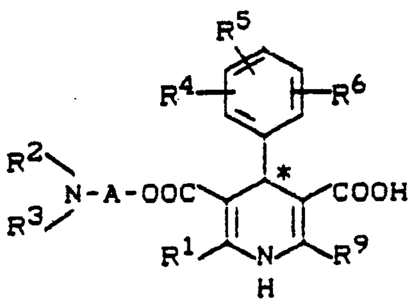

oder

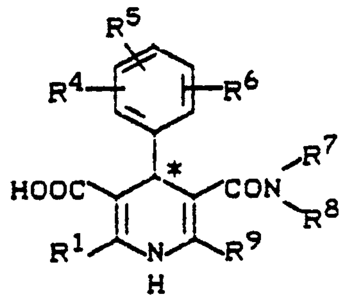

(IXa*) (IXb*)

herstellt, und die Verbindungen der allgemeinen Formel (IXa*) mit Verbindungen der allgemeinen Formel (Xa)

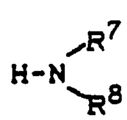

(Xa)

in welcher
$R^7$ und $R^8$ die oben angegebene Bedeutung haben
gegebenenfalls über aktivierte Säurederivate umsetzt
oder indem man
Verbindungen der allgemeinen Formel (IXb*) mit Verbindungen der allgemeinen Formel (Xb)

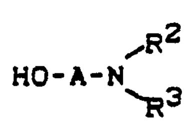

(Xb)

in welcher
$R^2$, $R^3$ und A die oben angegebene Bedeutung haben
gegebenenfalls über aktivierte Säurederivate umsetzt.

Je nach Art der verwendeten Ausgangsstoffe können die Synthesevarianten für die erfindungsgemäßen Verbindungen durch folgende Formelschemata wiedergegeben werden.

Als reaktive Säurederivate seien beispielsweise genannt; aktivierte Ester, Hydroxysuccinimidester, Säureimidazolide, Säurehalogenide, gemischte Anhydride oder die Umsetzung in Anwesenheit von Cyclohexylcarbodiimid.

Variante A

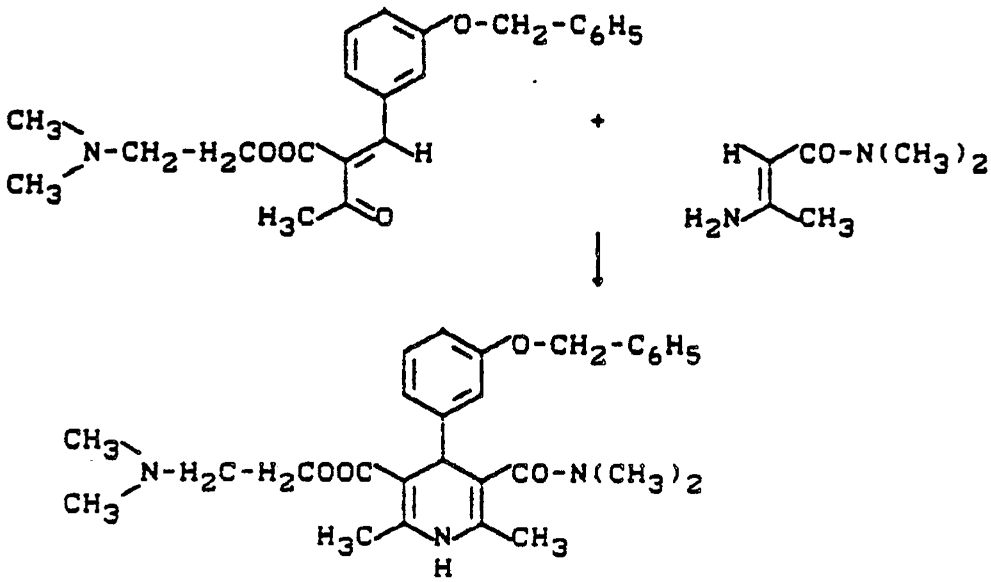

Variante B

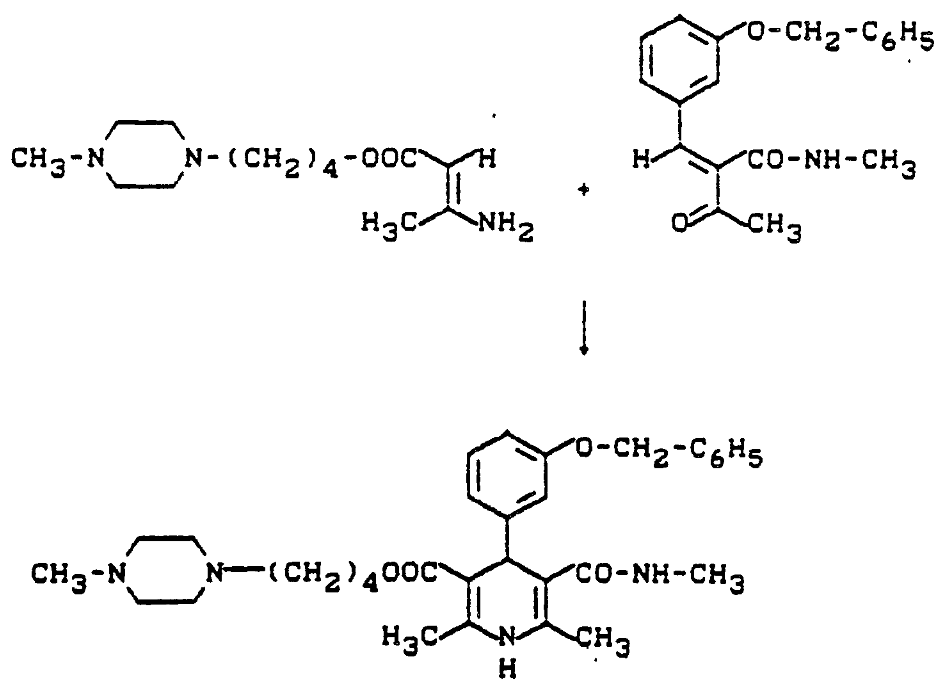

Variante C

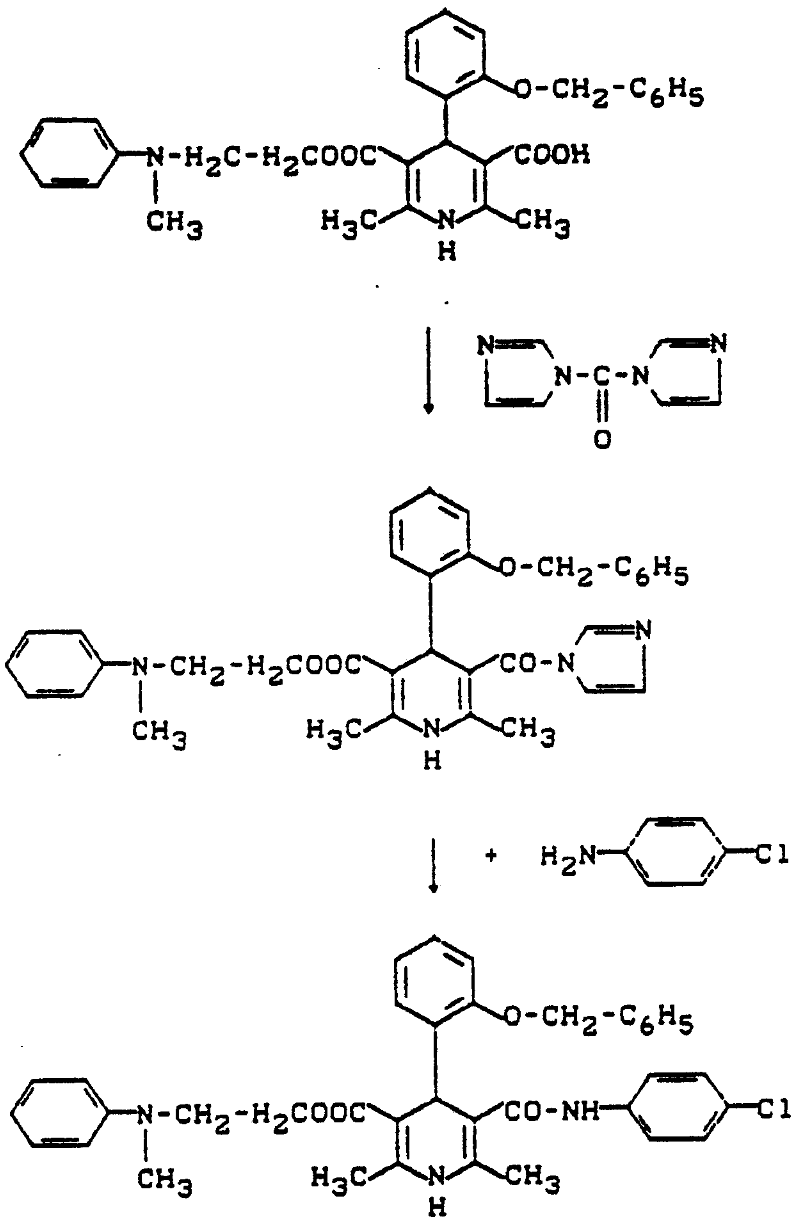

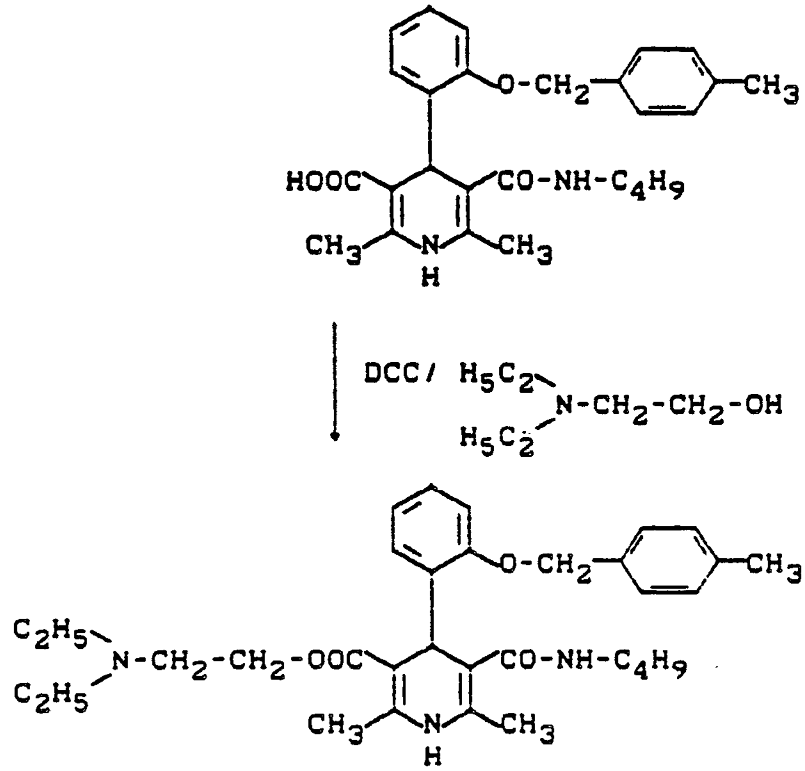


Für das Verfahren A und B gilt die Maßgabe, daß wenn $R^2$ und/oder $R^3$ Wasserstoff bedeuten, zunächst mit einer Aminoschutzgruppe, wie beispielsweise tert.-Butyloxycarbonyl oder Phthalimid blockiert wird und anschließend durch Abspaltung der Aminoschutzgruppe nach bekannter Methode die Verbindungen der allgemeinen Formel (I)

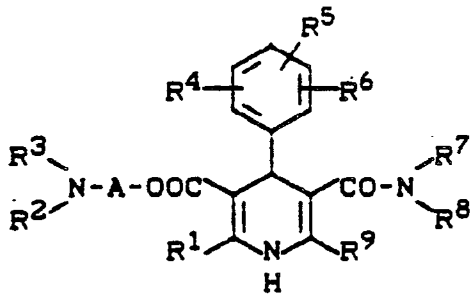

(I)

in welcher
$R^1$, $R^4$, $R^5$, $R^6$, $R^7$, $R^8$, $R^9$ und A die oben angegebene Bedeutung haben und $R^2$ und/oder $R^3$ Wasserstoff bedeuten,
dargestellt werden.

Verfahrensvarianten A - C

Als Lösemittel kommen Wasser oder alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A - C ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die als Ausgangsstoffe eingesetzten Aldehyde der allgemeinen Formel (II) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten β-Ketocarbonsäureester der allgemeinen Formel (III) sind bekannt oder können nach bekannten Methoden hergestellt werden [D. Borrmann in Houben Weyl's "Methoden der organischen Chemie" Bd. VII/4, 230 (1968); Y. Oikawa, K. Sugano, O. Yonemitsu, J. Org. Chem. 43, 2087 (1978)] [DOS 11 42 859].

Die als Ausgangsstoffe eingesetzten Enamine der allgemeinen Formeln (VI) und (VIII) sind bekannt oder können nach bekannten Methoden hergestellt werden [DOS 2 228 377] [F.A. Glickman, A.C. Cope, J. Am. Chem. Soc. 67, 1017 (1945)].

Die als Ausgangsstoffe eingesetzten Yliden-β-ketocarbonsäurederivate der allgemeinen Formeln (IV) und (VII) sind bekannt oder können nach bekannten Methoden hergestellt werden [G. Jones "The Knoevenagel Condensation" in Organic Reactions Bd. XV, 204 (1967)].

Die Durchführung der erfindungsgemäßen Verfahrensvariante C lehnt sich an die literaturbekannte Methodik zur Überführung von Carbonsäuren in Carbonsäureamide an. Dabei wird die Carbonsäure zunächst in eine aktivierte Form wie z.B. das Säurechlorid oder das Imidazolid überführt, die entweder als solche isoliert und in einem zweiten Reaktionsschritt umgesetzt werden, oder die in situ direkt zu den erfindungsgemäßen Verbindungen amidiert werden. Als aktivierende Reagenzien seien neben den anorganischen Halogeniden wie Thionylchlorid, Phosphortrichlorid oder Phosphorpentachlorid, oder dem Carbonyldiimidazol, Carbodiimide wie Cyclohexylcarbodiimid oder 1-Cyclohexyl-3-[2-(N-methyl-morpholino)ethyl]-carbodiimid-p-toluolsulfonat oder N-Hydroxy-phthalimid oder N-Hydroxy-benztriazol in Gegenwart von Dicyclohexylcarbodiimid beispielhaft erwähnt. Naturgemäß lassen sich die Dihydropyridinmonocarbonsäuren auch in Form ihrer Salze einsetzen. [Die Methodik der Amidierung wird beispielsweise beschrieben: Fieser & Fieser, Reagents for Organic Synthesis, John Wiley & Sons Inc. (1967), Seite 231 - 236; J.C. Shihan and G.P. Hess, J. Am. Chem. Soc. 77, 1067 (1955); U. Goodman, G.W. Kenner, Adv. in Protein Chem. 12, 488 (1957); W.A. Bonner, P.I. McNamee, J. Org. Chem. 26, 254 (1961); H.A. Staab, Angew. Chemie Int. Ed. 1, 351 (1962); Fieser & Fieser, Reagents for Organic Synthesis, John Wiley & Sons Inc. 1967, 116, 114; H.C. Beyerman, U.O. van der Brink, Re. Trav. 80, 1372 (1961); C.A. Buehler, D.E. Pearson, John Wiley & Sons, Volume I (1970), Seite 895 ff, Volume II, (1977)].

Als Lösemittel für Verfahrensvariante C kommen neben Wasser alle inerten organischen Lösemittel in Frage, die sich unter den Reaktionsbedingungen nicht verändern. Hierzu gehören vorzugsweise Ether wie Diethylether, Dioxan, Tetrahydrofuran, Glykolmonomethylether oder Glykoldimethylether, oder halogenierte Kohlenwasserstoffe wie Dichlormethan, Trichlormethan oder Tetrachlormethan, oder Amide wie Dimethylformamid Dimethylacetamid oder Hexamethylphosphorsäuretriamid, oder Kohlenwasserstoffe wie Benzol, Toluol oder Xylol, oder Acetonitril, Nitromethan, Pyridin, Dimethylsulfoxid oder Essigester. Ebenso können Gemische der genannten Lösemittel verwendet werden. Isoliert man die aktivierten Zwischenstufen der Dihydropyridinmonocarbonsäuren, so können auch die Amine der Formel (Xa) alleine als Verdünnungsmittel verwendet werden.

Die Reaktionstemperaturen können in einem breiten Bereich variiert werden. Im allgemeinen arbeitet man in einem Bereich von -70 °C bis +140 °C, bevorzugt von -20 °C bis +100 °C.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvariante C ist das Verhältnis der an der Reaktion beteiligten Stoffe beliebig. Im allgemeinen arbeitet man jedoch mit molaren Mengen der Reaktanden. Es hat sich jedoch als günstig erwiesen, das Amin in einem 5- bis 10-fach molaren Überschuß einzusetzen. Besonders zweckmäßig wird das Amin in einem großen Überschuß direkt als Lösemittel eingesetzt.

Die als Ausgangsstoffe eingesetzten Dihydropyridinmonocarbonsäuren der allgemeinen Formeln (IXa) und (IXb) sind nicht bekannt können aber nach bekannten Methoden hergestellt werden [DOS 2 847 236; 3

206 671; 2 962 241].

Die als Ausgangstoffe eingesetzten Amine der allgemeinen Formeln (Xa) und (Xb) sind bekannt oder können nach bekannten Methoden hergestellt werden [Houben Weyl's "Methoden der organischen Chemie" Bd XI/1; Paulsen, Angewandte Chemie 78, 501 - 566 (1966)].

Die Abspaltung der Aminoschutzgruppe erfolgt in an sich bekannter Weise unter sauren Bedingungen, wenn sie für den Phthalimidrest steht, erfolgt die Abspaltung der Schutzgruppe üblicherweise mit Hyrazinhydrat in organischen Lösemitteln wie Ethern, z.B. Tetrahydrofuran oder Dioxan, oder Alkoholen z.B. Methanol, Ethanol oder Isopropanol.

Die erfindungsgemäßen Verbindungen zeigen ein nicht vorhersehbares, wertvolles pharmakologisches Wirkspektrum. Sie beeinflussen die Kontraktionskraft des Herzens, den Tonus der glatten Muskulatur sowie den Elektrolyt- und Flüssigkeitshaushalt.

Sie können deshalb in Arzneimitteln zur Behandlung des pathologisch veränderten Blutdrucks und der Herzinsuffizienz, sowie als Koronartherapeutika eingesetzt werden.

Darüber hinaus können sie zur Behandlung von Herzrhythmusstörungen, Niereninsuffizienz, Leberzirrhose, Aszites, Lungenödem, Hirnödem, Schwangerschaftsödem, Glaukom oder Diabetes mellitus eingesetzt werden.

Die Herzwirkung der erfindungsgemäßen Verbindungen wurde am isolierten, stimulierten Papillarmuskel des Meerschweinchenherzens gefunden. Dazu wurden die Versuchstiere (200 g schwere Meerschweinchen beiderlei Geschlechts) getötet, der Thorax geöffnet und das Herz entnommen. Für die Versuche wurden anschließend jeweils möglichst kleine Papillarmuskeln aus der rechten Herzkammer herauspräpariert und horizontal in einem Organbad fixiert. Dabei wurde das eine Ende des Muskels durch zwei Metallelektroden gehalten, die gleichzeitig zur Reizung des Präparates dienten, während das andere Ende des Muskels über einen Faden mit einem Kraftaufnehmer verbunden war. Der Papillarmuskel wurde mit einer Frequenz von 1 Hz überschwellig gereizt. Das Organbad mit einem Volumen von ca. 2 ml wurde kontinuierlich mit einer Krebs-Henseleit-Lösung (Konzentration in mM: NaCl 118; $NaHCO_3$ 25; KCl 10; $KH_2PO_4$ 1.2; $MgSO_4$ 1.2; $CaCl_2$ 1.8; Glukose 10, pH 7.4) mit einer Geschwindigkeit von 4 ml/min bei einer Temperatur von 32 °C durchströmt. Die Kontraktionen des Papillarmuskels wurden isometrisch über den angeschlossenen Kraftaufnehmer gemessen und auf einem Schreiber registriert.

Die erfindungsgemäßen Stoffe wurden in der Krebs-Henseleit-Lösung in einer Konzentration von 10 µg/ml gegebenenfalls mit einem Lösungsvermittler (DMSO bis zu einer Konzentration von 0.5%) gelöst. Die erfindungsgemäßen Dihydropyridincarbonamide zeigten dabei bezogen auf die Kontrollwerte eine Hemmung der Kontraktionskraft des Papillarmuskels um mehr als 10%.

Zur Prüfung der renalen Wirkung wurden die Substanzen oral bei wachen männlichen Wistar-Ratten verabreicht. Nach Belastung mit physiologischer Kochsalzlösung wurde die Natriumausscheidung in Stoffwechselkäfigen gemessen.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Ge steinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Detergentien (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten

Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

Verfahrensvariante B

1,4-Dihydro-2,6-dimethyl-4-(2-benzyloxyphenyl)-pyridin-3-carbonsäure-2-(N-morpholino)ethylester-5-carbonsäure-cyclopropylamid-hydrochlorid

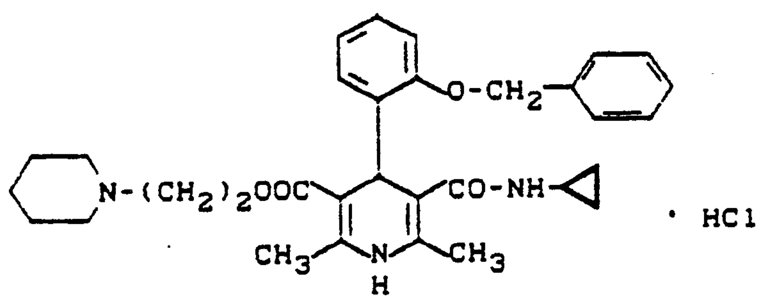

3,4 g (10 mmol) 2-Benzyloxybenzyliden-acetesigsäure-cyclopropylamid werden in 30 ml Isopropanol mit 2,1 g (10 mmol) β-Aminocrotonsäure-2-(N-morpholino)-ethylester 18 Stunden unter Argon gekocht und eingeengt. Der erhaltene ölige Rückstand wird über eine Kieselgelsäule mit Toluol/Aceton als Eluationsmittel gereinigt, die sauberen Fraktionen werden vereinigt und eingeengt. Der ölige Eindampfrückstand wird in Ether gelöst, mit HCl/Ether versetzt, eingeengt, zweimal mit Ethanol versetzt und eingeengt, mit Acetonitril verrührt, abgesaugt und mit Acetonitril gewaschen. Man erhält 1 g einer farblosen Substanz vom Schmelzpunkt 130° C unter Zersetzung.

Beispiel 2

Verfahrensvariante C

1,4-Dihydro-2,6-dimethyl-4-(2-(4-methylbenzyloxy)-phenyl)-pyridin-3-carbonsäure-(2-dimethylaminoethyl)-ester-5-carbonsäure-ethylamid-hydrochlorid

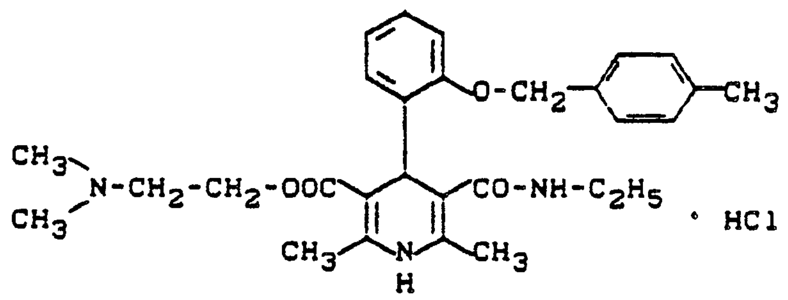

2 g (3,9 mmol) 1,4-Dihydro-2,6-dimethyl-4-[2-(4-methylbenzyloxy)-phenyl]-3-carbonsäure-(2-dimethylaminoethyl)-ester-5-carbonsäureimidazolid werden in 20 ml 50 %iger Ethylaminlösung 20 Stunden gerührt. Es wird eingeengt, in Essigester aufgenommen, 2 mal mit Wasser gewaschen, getrocknet und eingeengt. Die ölige Substanz wird durch Kieselgelchromatographie mit Toluol/Ethanol gereinigt. Die sauberen Fraktionen werden eingeengt und in das Hydrochlorid überführt. Man erhält 1,1 g farblose Kristalle vom Schmelzpunkt 147° C unter Zersetzung.

Beispiel 3

Verfahrensvariante A

1,4-Dihydro-2,6-dimethyl-4-(2-benzyloxyphenyl)-pyridin-3-carbonsäure-(2-N-benzyl-N-methylamino-ethyl)-ester-5-carbonsäure-propylamid

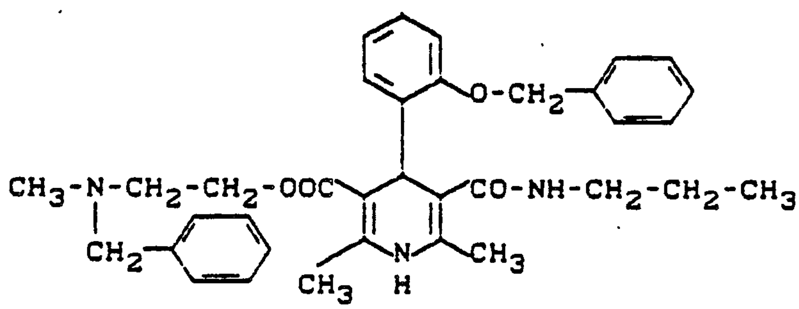

10 g (22,5 mmol) 2-Benzyloxy-benzyliden-acetessigsäure-(2-N-benzyl-N-methylamino-ethyl)-ester werden in 50 ml Isopropanol mit 3,2 g (22,5 mmol) $\beta$-Aminocrotonsäurepropylamid 4 Stunden unter Argon gekocht. Es wird eingeengt, in Essigester aufgenommen, 2 mal mit Wasser gewaschen, getrocknet und eingeengt. Der Eindampfrückstand wird über eine Kieselgelsäule mit Toluol/Essigester 1:1 gereinigt. Die reinen Fraktionen werden durch Verrühren mit Ether kristallisiert. Man erhält 5,5 g Kristalle vom Schmelzpunkt 100° C.

| Beispiel Nr. | Formel | Schmelzpunkt ($R_F$-Wert*) | Verf. |
|---|---|---|---|
| 4 | 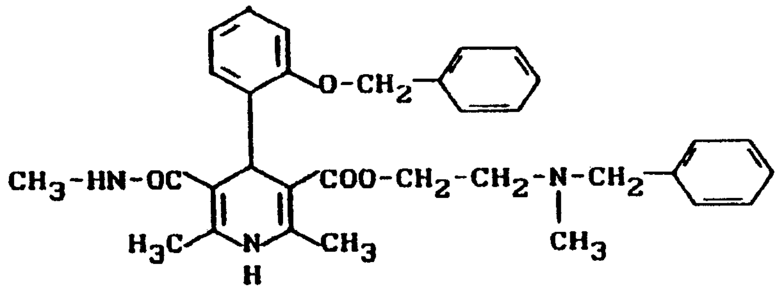 | (1) 0,46 | C |
| 5 | 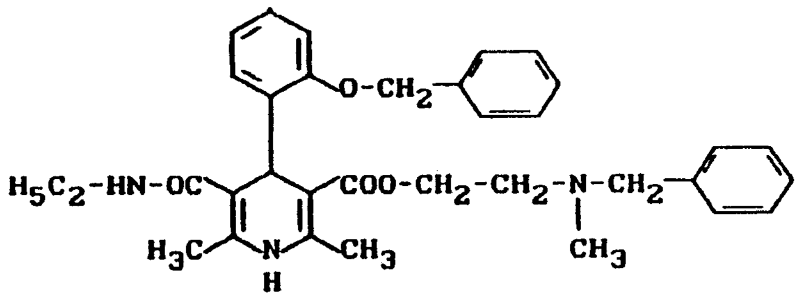 | (1) 0,51 | C |
| 6 | 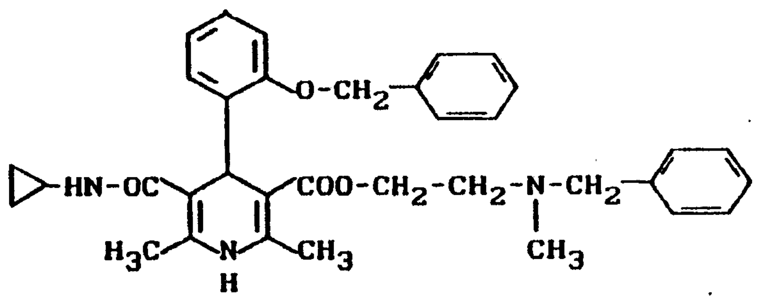 | (1) 0,5 | C |

* $R_F$-Werte Laufmittel: (1) Toluol:Aceton (1:1)
DC Fertigplatten Merck, Kieselgel 60 F 254

| Beispiel Nr. | Formel | Schmelzpunkt ($R_F$-Wert*) | Verf. |
|---|---|---|---|
| 7 | 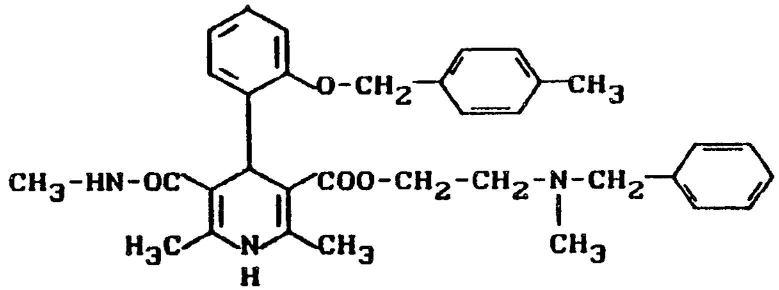 | (1) 0,5 | C |
| 8 | 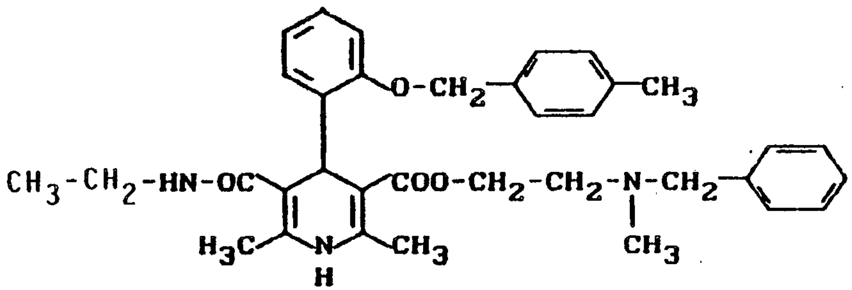 | (1) 0,53 | C |
| 9 | 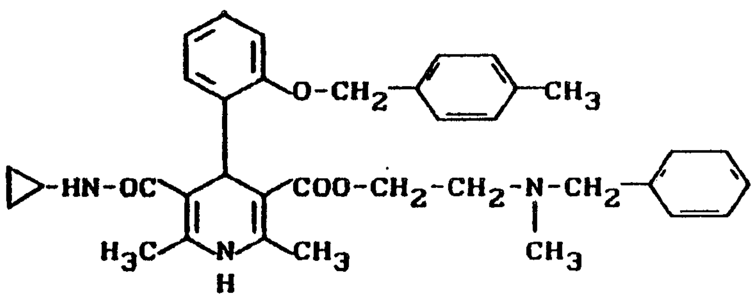 | (1) 0,52 | C |

| Beispiel Nr. | Formel | Schmelzpunkt ($R_F$-Wert*) | Verf. |
|---|---|---|---|
| 10 | 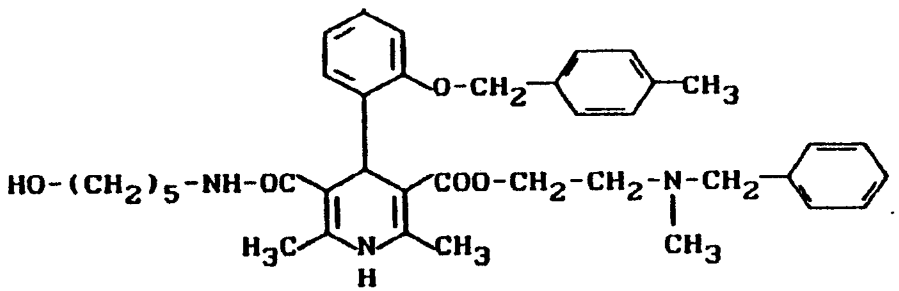 | (1) 0,26 | C |
| 11 | 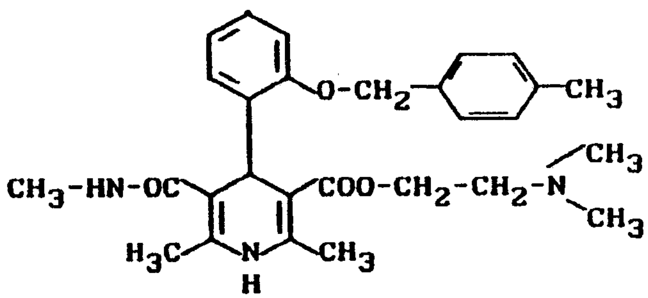 | 90-95° C | C |
| 12 | 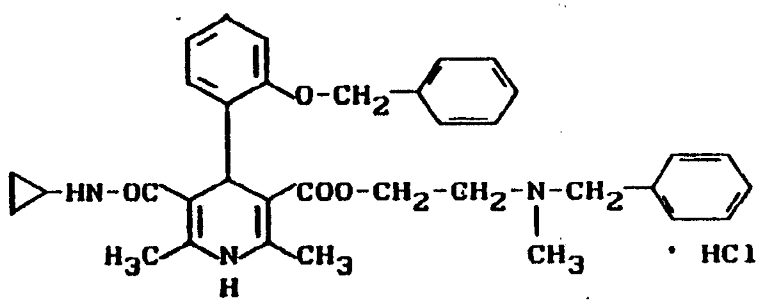 | 159° C | A |

| Beispiel Nr. | Formel | Schmelzpunkt ($R_F$-Wert*) | Verf. |
|---|---|---|---|
| 13 | 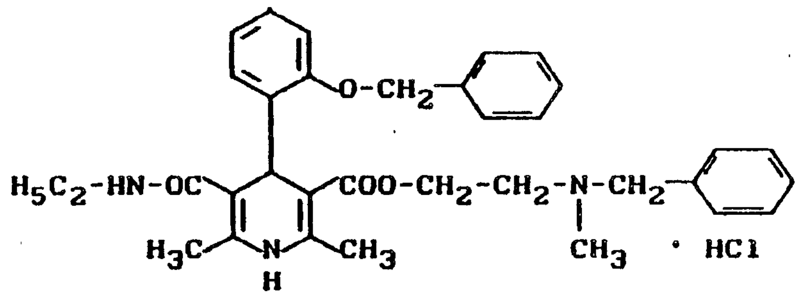 | 143° C | A |
| 14 | 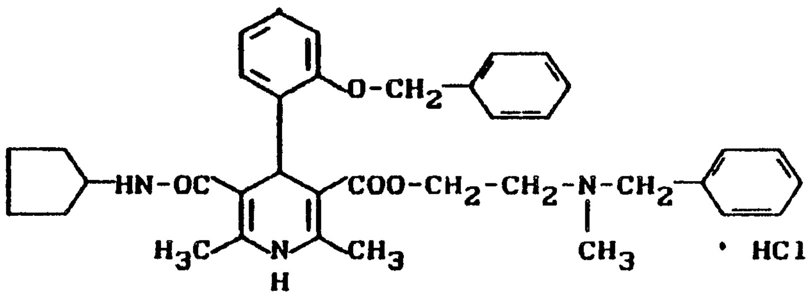 | 120° C | A |
| 15 | 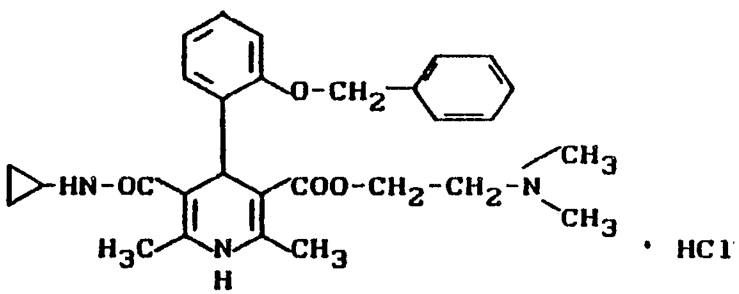 | 155° C Z | B |

| Beispiel Nr. | Formel | Schmelzpunkt ($R_F$-Wert*) | Verf. |
|---|---|---|---|
| 16 | 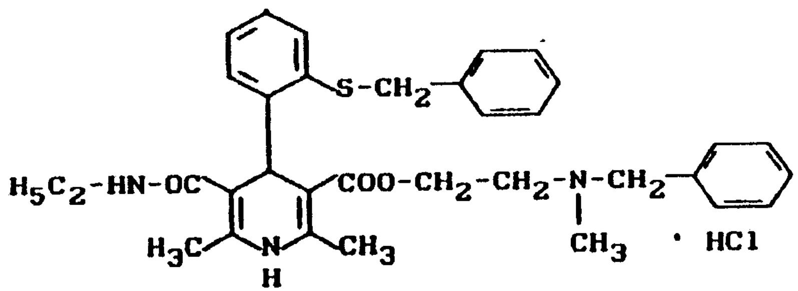 | 145-148° C Z. | A |
| 17 | 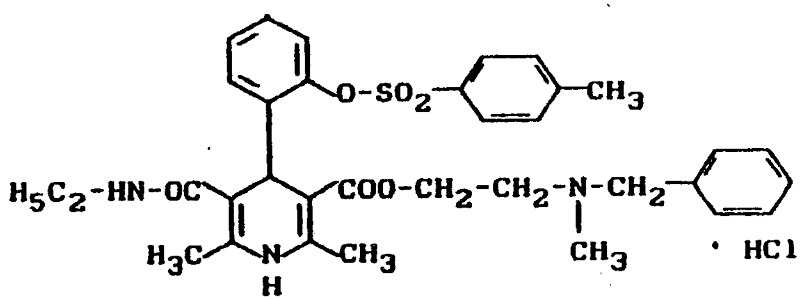 | 225° C Z. | A |
| 18 | 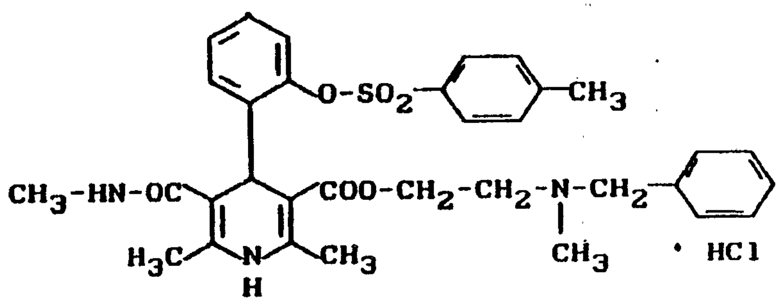 | 177-180° C | A |

| Beispiel Nr. | Formel | Schmelzpunkt ($R_F$-Wert*) | Verf. |
|---|---|---|---|
| 19 | 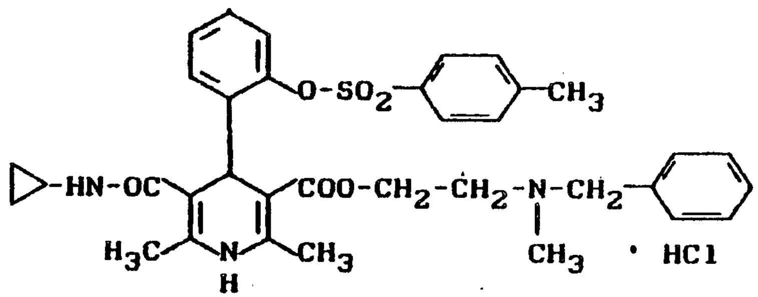 | 237-239° C Z. | A |
| 20 | 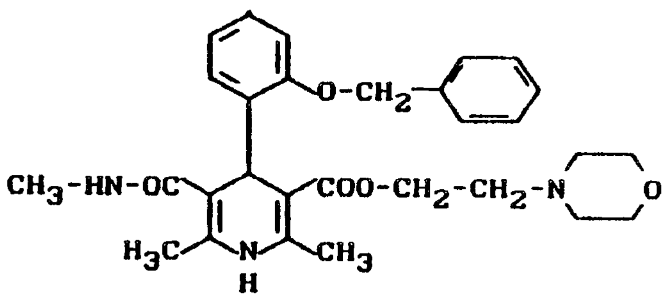 | 0,25 | A |
| 21 | 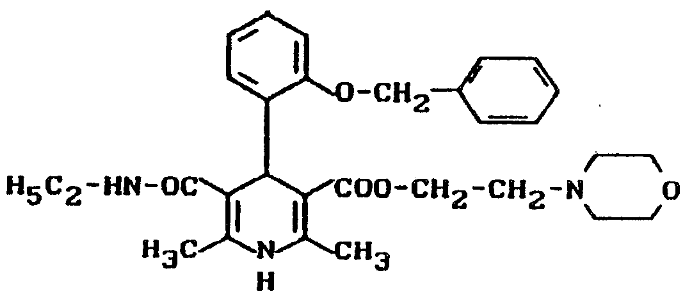 | 0,31 | A |

| Beispiel Nr. | Formel | Schmelzpunkt ($R_F$-Wert*) | Verf. |
|---|---|---|---|
| 22 | 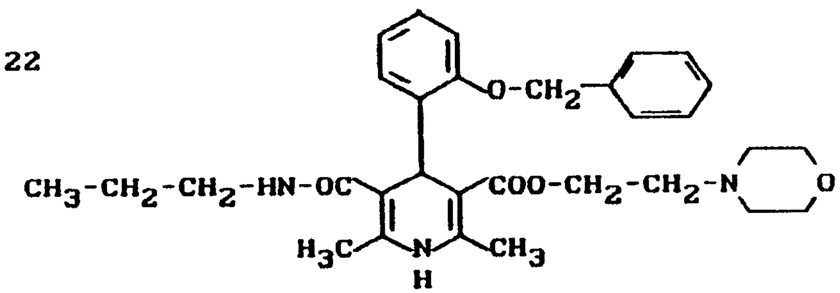 | 0,35 | A |
| 23 | 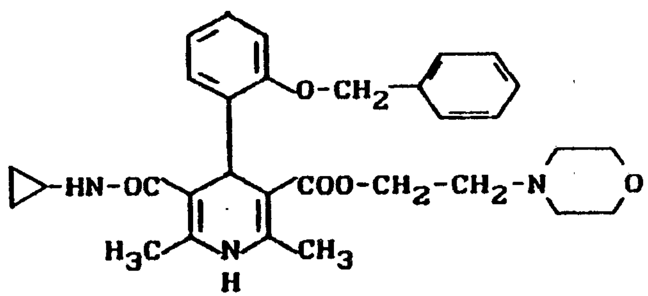 | 0,29 | A |
| 24 | 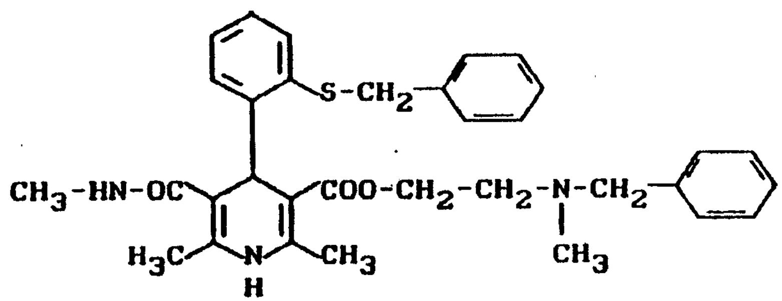 | 0,41 | A |

| Beispiel Nr. | Formel | Schmelzpunkt ($R_F$-Wert*) | Verf. |
|---|---|---|---|
| 25 | 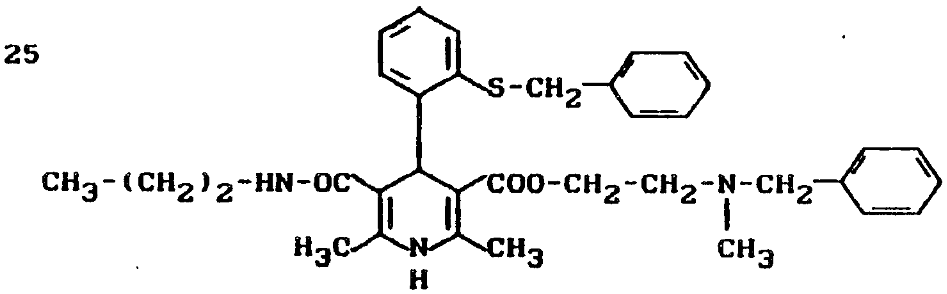 | 0,48 | A |
| 26 | 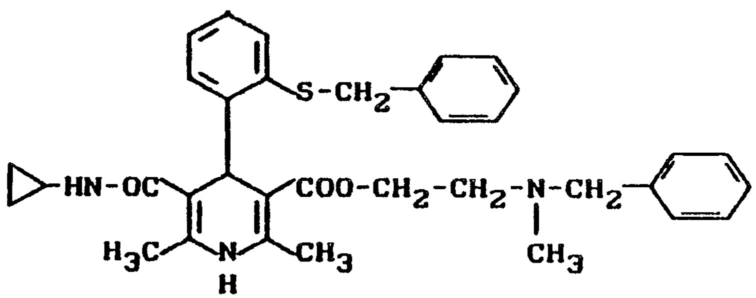 | 84-86° C | A |
| 27 | 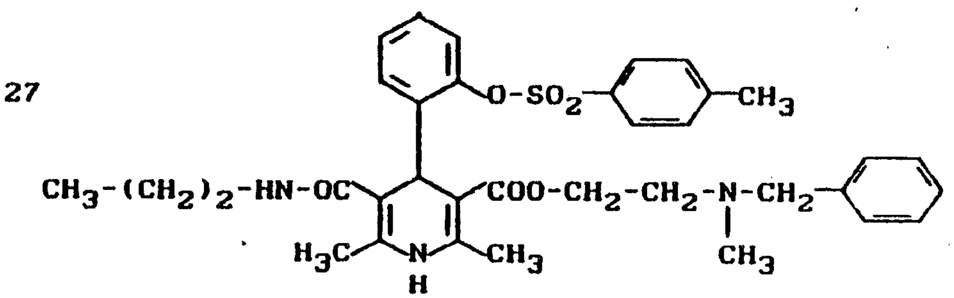 | 0,42 | A |

| Beispiel Nr. | Formel | Schmelzpunkt ($R_F$-Wert*) | Verf. |
|---|---|---|---|
| 28 | 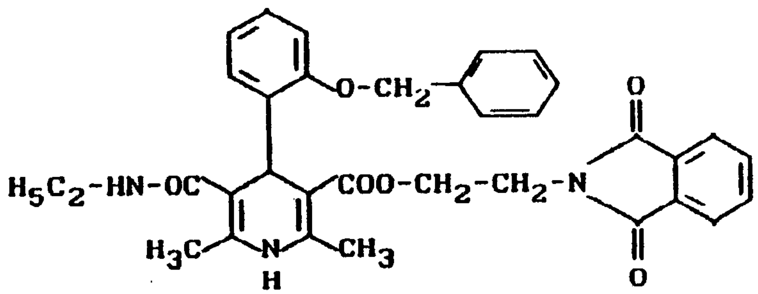 | (2) 0,77 | A |
| 29 | 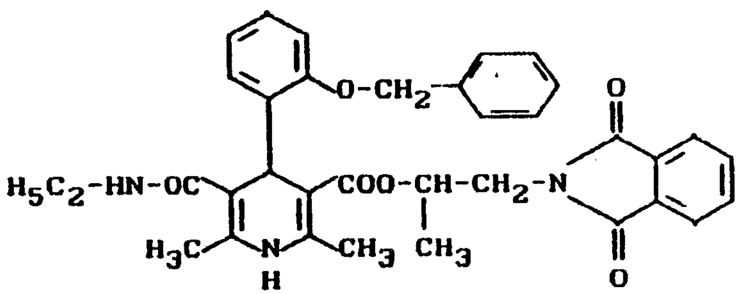 | (2) 0,74 | A |
| 30 | 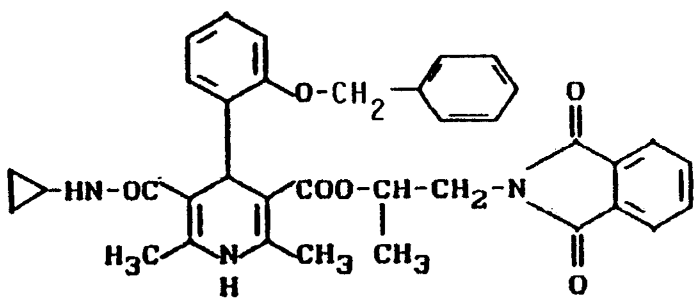 | (2) 0,72 | A |

| Beispiel Nr. | Formel | Schmelzpunkt ($R_F$-Wert*) | Verf. |
|---|---|---|---|
| 31 | 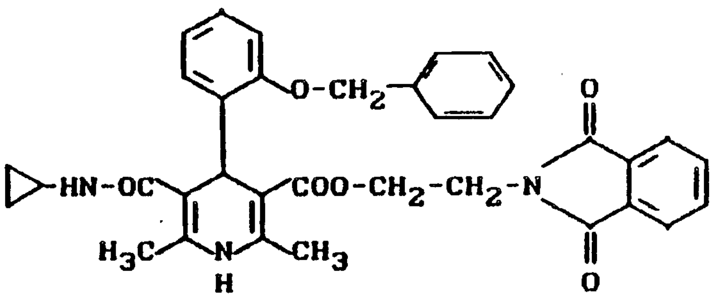 | 194 - 197° C<br>(2) 0,63 | A |

* $R_f$-Werte (Laufmittel): (2) = Methylenchlorid/Methanol (10:1) HPTLC-Fertigplatten, Kieselgel 60 F 254

Beispiel 32

4-(2-Benzyloxyphenyl)-1,4-dihydro-2,6-dimethyl-pyridin-3-carbonsäure-(2-aminomethyl)-ester-5-

carbonsäureethylamid

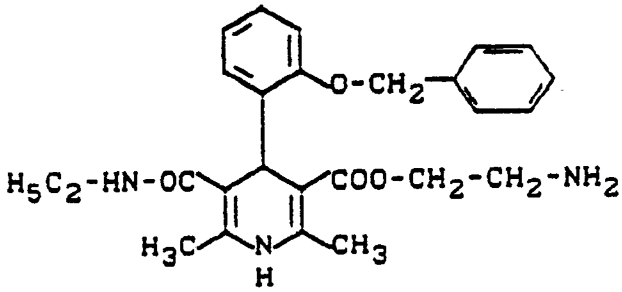

Eine Lösung von 11,0 g (18,97 mmol) 4-(2-Benzyloxyphenyl)-1,4-dihydro-2,6-dimethyl-pyridin-3-carbonsäure-(2-phthalimido-ethyl)-ester-5-carbonsäureethylamid (Beispiel 28) und 95,0 mmol Hydrazinhydrat werden in 100 ml Ethanol 2 h unter Rückfluß gekocht. Danach wird die Lösung abgekühlt und der Rückstand filtriert. Dieser wird mit Methylenchlorid nachgewaschen und das Filtrat im Vakuum eingeengt. Dann wird der eingeengte Rückstand 1 mal mit einer 2 N Lösung Kaliumhydroxid und anschließend 3 mal mit Wasser gewaschen. Das Produkt wird auf einer Kieselgelsäule mit Methylenchlorid/Methanol-Gemischen gereinigt

Ausbeute: 6,56 g (76,9% der Theorie)

Das Produkt ist amorph.

Die in der folgenden Tabelle aufgeführten Beispiele wurden analog der Vorschrift zu Beispiel 32 hergestellt.

| Beispiel Nr. | Formel | Schmelzpunkt ($R_F$-Wert*) |
|---|---|---|
| 33 | 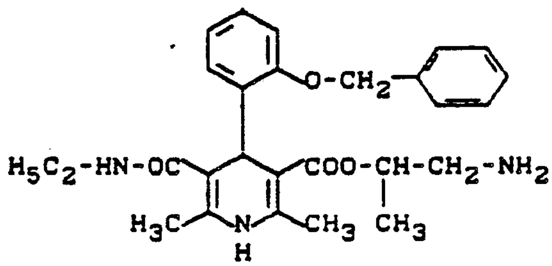 | (3) 0,66 |
| 34 | 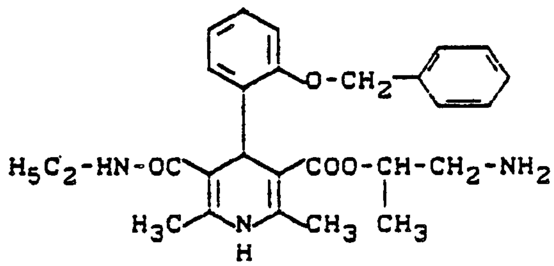 | (3) 0,61 |
| 35 | 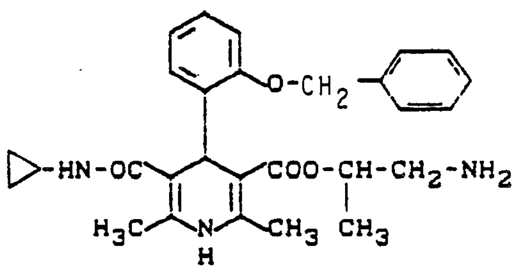 | (3) 0,67 |
| 36 | 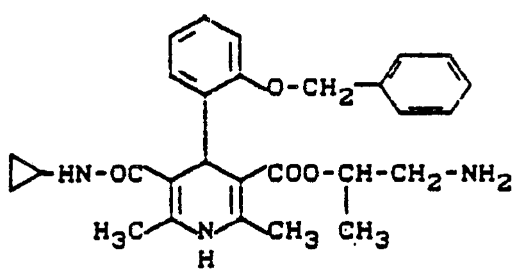 | (3) 0,64 |

| Beispiel Nr. | Formel | Schmelzpunkt ($R_F$-Wert*) |
|---|---|---|
| 37 | 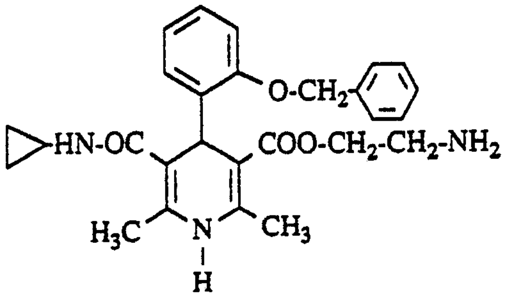 | 179-181°C |

*$R_F$-Werte Laufmittel: (3) Methylenchlorid/Methanol (5:1)

| Beispiel Nr. | Formel | Schmelzpunkt ($R_F$-Wert*) |
|---|---|---|
| 38 | 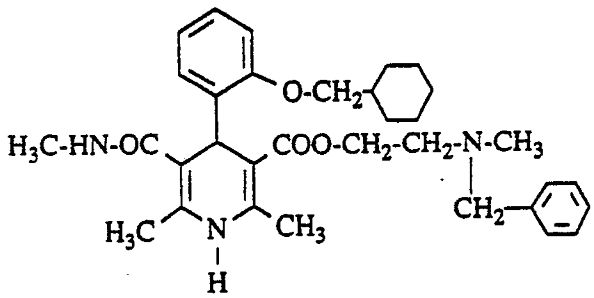 | 140-144°C |
| 39 | 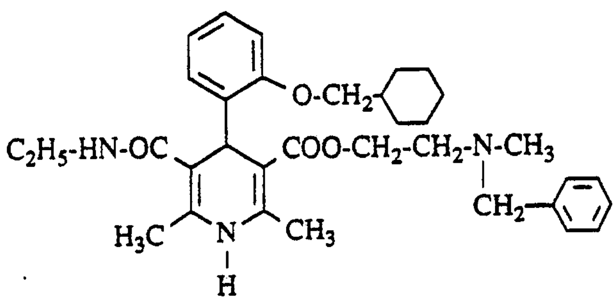 | (4) 0,54 |
| 40 | 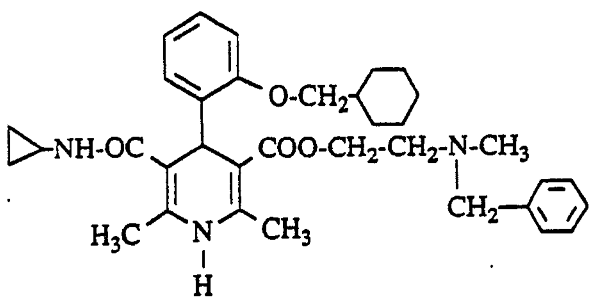 | (4) 0,54 |

| Beispiel Nr. | Formel | Schmelzpunkt ($R_F$-Wert*) |
|---|---|---|
| 41 | 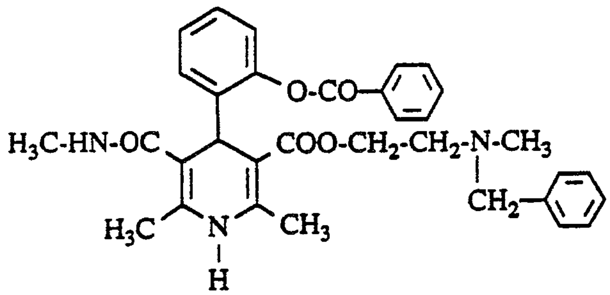 | (4) 0,35 |
| 42 | 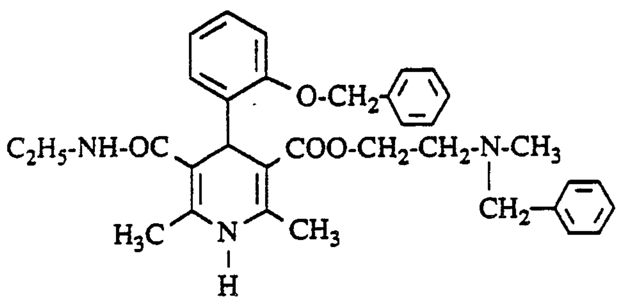 -Enantiomer | 112°C |
| 43 | 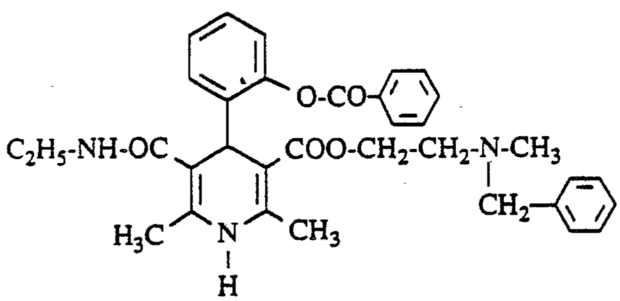 | (4) 0,40 |
| 44 | 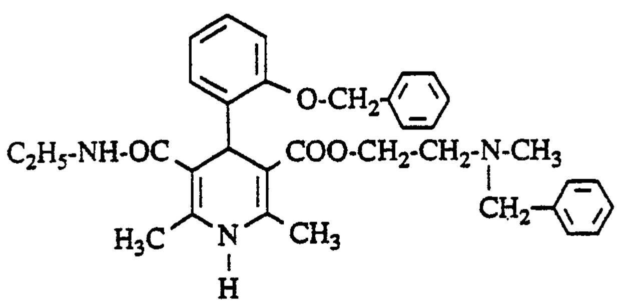 +Enantiomer | 110°C |

| Beispiel Nr. | Formel | Schmelzpunkt ($R_F$-Wert*) |
|---|---|---|
| 45 | 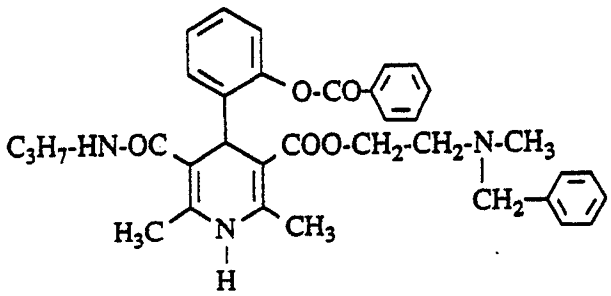 | (4) 0,42 |

*$R_F$-Werte Laufmittel: (4) Toluol/Aceton 1:1

## Ansprüche

1. Dihydropyridinamide der allgemeinen Formel (I)

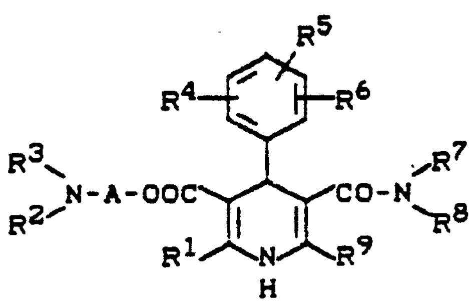

(I)

in welcher
$R^1$ und $R^9$ gleich oder verschieden sind und
- für geradkettiges, verzweigtes oder cyclisches Alkyl mit bis zu 6 Kohlenstoffatomen stehen, das gegebenenfalls durch Hydroxy, Cyano, Phenyl oder Halogen substituiert ist, oder
- für Cyano oder Phenyl stehen,

$R^2$ und $R^3$ gleich oder verschieden sind und für Wasserstoff, für geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, durch Phenyl, welches gegebenenfalls durch Nitro, Phenyl, Cyano, Trifluormethyl, Trifluormethoxy, Alkyl mit bis zu 4 Kohlenstoffatomen, Halogen oder Alkoxy mit bis zu 4 Kohlenstoffatomen substituiert ist, für Cycloalkyl mit 3 bis 8 Kohlenstoffatomen oder
- für Aryl mit 6 bis 12 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe, Acetylamino oder Benzoylamino substituiert sein kann, oder

$R^2$ und $R^3$ zusammen einen 5- bis 7-gliedrigen gesättigten oder ungesättigten heterocyclischen Ring bilden, der als Heteroatom ein Sauerstoffatom, ein Schwefelatom oder ein zusätzliches Stickstoffatom enthalten kann, welches gegebenenfalls durch eine Gruppe $R^{13}$ substituiert sein kann, die für Wasserstoff, eine geradkettige oder verzweigte, gesättigte oder ungesättigte Alkylgruppe mit bis zu 10 Kohlenstoffatomen

steht, die gegebenenfalls durch Phenyl, welches durch Halogen, Alkyl mit bis zu 4 C-Atomen, Alkoxy mit bis zu 4 C-Atomen, Nitro und Halogenalkyl mit bis zu 2 C-Atomen substituiert sein kann oder für Phenyl steht, das gegebenenfalls durch Halogen, Cyano, Nitro, Alkyl mit bis zu 2 C-Atomen, Alkoxy mit bis zu 2 C-Atomen oder Halogenalkyl mit bis zu 2 C-Atomen substituiert sein kann,

$R^4$ und $R^5$ gleich oder verschieden sind und

- für Wasserstoff, Halogen, Alkyl mit bis zu 6 Kohlenstoffatomen, Alkoxy mit bis zu 6 Kohlenstoffatomen, Alkylthio mit bis zu 4 Kohlenstoffatomen, Cyano, Nitro, Dialkylamino mit jeweils bis zu 4 Kohlenstoffatomen je Alkylgruppe, Trifluormethyl, Trifluormethoxy, Difluormethoxy oder Trifluormethylthio stehen,

$R^6$ - für eine Gruppe der Formel $-O-(CH_2)n-R^{10}$, $-S-(CH_2)_n-R^{10}$, $-O-SO_2-(CH_2)_n-R^{10}$, $-O-CO-(CH_2)_n-R^{10}$ steht, worin

n - 0 bis 4 bedeutet,

und

$R^{10}$- Cyclohexyl bedeutet oder Aryl mit 6 bis 10 Kohlenstoffatomen bedeutet, das bis zu 4-fach gleich oder verschieden durch Halogen, Cyano, Nitro, Trifluormethyl, Trifluormethoxy, Difluormethoxy, Trifluormethylthio, Alkyl mit bis zu 6 Kohlenstoffatomen, Alkoxy mit bis zu 6 Kohlenstoffatomen, Alkylthio mit bis zu 4 Kohlenstoffatomen, Amino, Alkylamino mit bis zu 6 Kohlenstoffatomen, Dialkylamino mit jeweils bis zu 6 Kohlenstoffatomen je Alkylgruppe oder Acetylamino substituiert sein kann oder

- einen 5- bis 7-gliedrigen gesättigten oder ungesättigten heterocyclischen Ring bedeutet, der als Heteroatome ein Sauerstoffatom, ein Schwefelatom oder zwei Stickstoffatome enthalten kann,

und

$R^7$ und $R^8$ gleich oder verschieden sind und jeweils

- für Wasserstoff,

- für Cycloalkyl mit 3 bis 8 Kohlenstoffatomen oder

- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^{11}R^{12}$ substituiert sind,

worin

$R^{11}$ und $R^{12}$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 10 Kohlenstoffatomen, Acetyl, Benzoyl, Alkylsulfonyl mit bis zu 6 Kohlenstoffatomen oder Phenylsulfonyl bedeuten, oder $R^7$ und $R^8$ jeweils

- für Aryl mit 6 bis 10 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, Alkylamino mit bis zu 4 Kohlenstoffatomen, Dialkylamino mit bis zu 4 Kohlenstoffatomen je Alkylgruppe, Acetylamino oder Benzoylamino substituiert ist, oder

- für einen 5- bis 7-gliedrigen gesättigten oder ungesättigten heterocyclischen Ring stehen, der als zusätzliches Heteroatom ein Sauerstoffatom, ein Schwefelatom oder eine NH- oder N-Alkyl-Gruppe (1-4 C-Atome) enthalten kann, und

A für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^{14}$ unterbrochen sein kann, in der

$R^{14}$ Wasserstoff, Alkyl bis zu 4 Kohlenstoffatomen, Benzyl oder Phenethyl bedeuten kann,

und/oder

der substituiert sein kann durch Halogen, Hydroxy, Acetoxy, Carboxy, Alkoxycarbonyl mit bis zu 8 Kohlenstoffatomen oder Phenyl, das gegebenenfalls substituiert sein kann durch Halogen, Alkyl mit bis zu 2 Kohlenstoffatomen, Alkoxy mit bis zu 2 Kohlenstoffatomen, Halogenmethyl, Halogenmethoxy, Hydroxy oder Cyano,

und deren physiologisch unbedenklichen Salze, in Form ihrer Diastereomerengemische, Racemformen und Antipoden.

2. Verbindungen der allgemeinen Formel (I) gemäß Anspruch 1, in welcher

$R^1$ und $R^9$ gleich oder verschieden sind und jeweils

- für geradkettiges oder verzweigtes Alkyl mit bis zu 4 Kohlenstoffatomen stehen, das gegebenenfalls durch Hydroxy oder Phenyl substituiert ist,

$R^2$ und $R^3$ gleich oder verschieden sind und für Wasserstoff, für geradkettiges oder verzweigtes Alkyl,

Alkenyl oder Alkinyl mit jeweils bis zu 8 Kohlenstoffatomen stehen, das substituiert sein kann, durch Halogen, Hydroxy, Alkoxy mit bis zu 4 Kohlenstoffatomen, Cyano, Trifluormethyl, Alkylthio mit bis zu 4 Kohlenstoffatomen, Alkylcarbonyl mit bis zu 4 Kohlenstoffatomen im Alkylrest, Carboxy oder Alkoxycarbonyl mit bis zu 8 Kohlenstoffatomen, durch Phenyl, welches gegebenenfalls durch Nitro, Phenyl, Trifluormethyl, Trifluormethoxy, Alkyl mit bis zu 2 Kohlenstoffatomen, Halogen oder Alkoxy mit bis zu 2 Kohlenstoffatomen substituiert ist, für Cycloalkyl mit 3 bis 8 Kohlenstoffatomen oder

- für Aryl mit 6 bis 12 Kohlenstoffatomen stehen, das bis zu 2-fach gleich oder verschieden durch Nitro, Cyano, Halogen, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Difluormethoxy, Trifluormethylthio, Amino, Alkylamino mit bis zu 2 Kohlenstoffatomen, Dialkylamino mit jeweils bis zu 2 Kohlenstoffatomen je Alkylgruppe, Acetylamino oder Benzoylamino substituiert sein kann,

oder

$R^2$ und $R^3$ für einen 5- bis 7-gliedrigen gesättigten oder ungesättigten heterocyclischen Ring bilden, der als Heteroatom ein Sauerstoffatom, ein Schwefelatom oder ein zusätzliches Stickstoffatom enthalten kann, welches gegebenenfalls durch eine Gruppe $R^{13}$ substituiert sein kann, die für Wasserstoff, eine geradkettige oder verzweigte, gesättigte oder ungesättigte Alkylgruppe mit bis zu 10 Kohlenstoffatomen steht, die gegebenenfalls durch Phenyl, welches durch Halogen, Alkyl mit bis zu 4 C-Atomen, Alkoxy mit bis zu 4 C-Atomen, Nitro und Halogenalkyl mit bis zu 2 C-Atomen substituiert sein kann oder für Phenyl steht, das gegebenenfalls durch Halogen, Cyano, Nitro, Alkyl mit bis zu 2 C-Atomen, Alkoxy mit bis zu 2 C-Atomen oder Halogenalkyl mit bis zu 2 C-Atomen substituiert sein kann,

$R^4$ und $R^5$ gleich oder verschieden sind und

- für Wasserstoff, Fluor, Chlor, Brom, Alkyl mit bis zu 4 Kohlenstoffatomen, Alkoxy mit bis zu 4 Kohlenstoffatomen, Methylthio, Cyano, Nitro, Trifluormethyl oder Trifluormethoxy stehen,

$R^6$ - für eine Gruppe der Formel $-O-(CH_2)n-R^{10}$, $-S-(CH_2)_n-R^{10}$, $-O-SO_2-(CH_2)_n-R^{10}$ oder $-O-CO-(CH_2)_n-R^{10}$ steht,

worin

n - 0 bis 3 bedeutet,

und

$R^{10}$- Cyclohexyl oder Phenyl bedeutet, das bis zu 3-fach gleich oder verschieden substituiert sein kann durch Fluor, Chlor, Brom, Cyano, Nitro, Trifluormethyl, Trifluormethoxy, Alkyl mit bis zu 4 Kohlenstoffatomen, Alkoxy mit bis zu 4 Kohlenstoffatomen, Methylthio, Amino, Alkylamino mit bis zu 4 Kohlenstoffatomen, Dialkylamino mit jeweils bis zu 4 Kohlenstoffatomen je Alkylgruppe, Acetylamino, oder

- Pyridyl, Thienyl, Furyl, Pyrimidyl oder Pyrazinyl bedeutet,

und

$R^7$ und $R^8$ gleich oder verschieden sind und jeweils

- für Wasserstoff, Cycloalkyl mit 3 bis 7 Kohlenstoffatomen stehen oder

- für geradkettiges oder verzweigtes Alkyl oder Alkenyl mit bis zu 14 Kohlenstoffatomen stehen, die substituiert sein können durch Fluor, Chlor, Brom, Hydroxy, Alkoxy mit bis zu 6 Kohlenstoffatomen, Alkylthio mit bis zu 6 Kohlenstoffatomen, Alkylcarbonyl mit bis zu 6 Kohlenstoffatomen im Alkylrest, Carboxy, Alkoxycarbonyl mit bis zu 6 Kohlenstoffatomen, durch gegebenenfalls durch Nitro, Trifluor methyl, Methyl oder Methoxy substituierte Phenyl- oder Phenoxy-Rest oder wobei die Reste Alkyl und Alkenyl gegebenenfalls substituiert sind durch Cyano und/oder durch eine Gruppe der Formel $-NR^{11}R^{12}$,

worin

$R^{11}$ und $R^{12}$ gleich oder verschieden sind und jeweils Wasserstoff, Alkyl mit bis zu 6 Kohlenstoffatomen, Benzyl, Phenethyl, Phenyl, Acetyl, Benzoyl, Alkylsulfonyl mit bis zu 4 Kohlenstoffatomen oder Phenylsulfonyl bedeuten,

oder

- für Phenyl oder Naphthyl stehen, die bis zu 3-fach gleich oder verschieden substituiert sein können durch Nitro, Fluor, Chlor, Brom, Alkyl mit bis zu 4 Kohlenstoffatomen, Alkoxy mit bis zu 4 Kohlenstoffatomen, Alkylthio mit bis zu 4 Kohlenstoffatomen, Trifluormethyl, Trifluormethoxy, Amino, Alkylamino mit bis zu 6 Kohlenstoffatomen, Dialkylamino mit jeweils bis zu 6 Kohlenstoffatomen je Alkylgruppe, Acetylamino oder durch Benzoylamino, oder

- für Pyrrolidino, Pyridino, Morpholino oder für Piperazino, $N-C_1-C_4$-Alkylpiperazino, $N-C_7-C_9$-Aralkyl- oder N-Phenyl-piperazino stehen,

und

A für einen geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^{14}$ unterbrochen sein kann, in der

$R^{14}$ Wasserstoff, Alkyl bis zu 2 Kohlenstoffatomen, Benzyl oder Phenethyl bedeuten kann,
und/oder
der substituiert sein kann durch Halogen, Hydroxy, Acetoxy, Carboxy, Alkoxycarbonyl mit bis zu 4 Kohlenstoffatomen oder durch Phenyl, das gegebenenfalls substituiert sein kann durch Halogen, Alkyl mit bis zu 2 Kohlenstoffatomen, Alkoxy mit bis zu 2 Kohlenstoffatomen, Trifluormethyl oder Trifluormethoxy
sowie deren physiologisch unbedenklichen Salze.

3. Verbindungen der allgemeinen Formel (I) gemäß Anspruch 1, in welcher
$R^1$ und $R^9$ gleich oder verschieden sind und für Methyl, Ethyl oder Benzyl stehen,
$R^2$ und $R^3$ gleich oder verschieden sind und für Wasserstoff, für geradkettiges oder verzweigtes Alkyl, Alkenyl mit bis zu 6 Kohlenstoffatomen stehen, das substituiert sein kann durch Hydroxy, Carboxy, Alkoxycarbonyl mit bis zu 4 C-Atomen im Alkylrest, durch Phenyl, welches gegebenenfalls durch Nitro, Halogen, Trifluormethyl, Trifluormethoxy, Methyl oder Methoxy substituiert ist, für Cycloalkyl mit 3 bis 6 Kohlenstoffatomen oder
- für Phenyl stehen, das bis zu 2-fach gleich oder verschieden durch Nitro, Halogen, Alkyl mit bis zu 2 Kohlenstoffatomen, Alkoxy mit bis zu 2 Kohlenstoffatomen, Alkylthio mit bis zu 2 Kohlenstoffatomen, Trifluormethyl, Trifluormethoxy, Difluormethoxy, Trifluormethylthio, Amino, Alkylamino mit bis zu 2 Kohlenstoffatomen oder Dialkylamino mit jeweils bis zu 2 Kohlenstoffatomen je Alkylgruppe substituiert sein kann,
oder
- für einen 5- bis 7-gliedrigen gesättigten oder ungesättigten heterocyclischen Ring stehen, der als Heteroatom ein Sauerstoffatom, ein Schwefelatom oder ein zusätzliches Stickstoffatom enthalten kann, welches gegebenenfalls durch eine Gruppe $R^{13}$ substituiert sein kann, die Wasserstoff, Alkyl mit bis zu 4 C-Atomen, Benzyl, Phenethyl oder gegebenenfalls durch Halogen, Methyl oder Methoxy substituiertes Phenyl bedeutet,
$R^4$ und $R^5$ gleich oder verschieden sein können und jeweils für Wasserstoff, Fluor, Chlor, Methyl, Ethyl, Methoxy, Ethoxy, Nitro oder Trifluormethyl stehen,
$R^6$ für eine Gruppe der Formel $-O-(CH_2)_nR^{10}$, $-S-(CH_2)_nR^{10}$, $-O-SO_2-(CH_2)_n-R^{10}$ oder $-O-CO-(CH_2)_n-R^{10}$ steht,
worin
n 0 bis 2 bedeutet und
$R^{10}$- Cyclohexyl oder Phenyl bedeutet, das bis zu 2-fach gleich oder verschieden substituiert sein kann durch Fluor, Chlor, Nitro, Trifluormethyl, Methyl, Methoxy, Amino, Methylamino, Dimethylamino, Ethylamino, Diethylamino oder Acetylamino, oder
- eine α-, β- oder eine γ-Pyridylgruppe bedeutet,
$R^7$ - für Wasserstoff oder Alkyl mit bis zu 4 Kohlenstoffatomen steht
und
$R^8$ - für Wasserstoff, Cyclopropyl, Cyclopentyl, Cyclohexyl steht, oder
- für geradkettiges oder verzweigtes Alkyl oder Alkenyl mit bis zu 10 Kohlenstoffatomen steht, das substituiert sein kann durch Fluor, Chlor, Hydroxy, Alkoxy mit bis zu 4 Kohlenstoffatomen, Alkylthio mit bis zu 4 Kohlenstoffatomen, Alkylcarbonyl mit bis zu 4 Kohlenstoffatomen im Alkylrest, Carboxy, Alkoxycarbonyl mit bis zu 4 Kohlenstoffatomen, Phenoxy, Phenyl oder die obengenannten Reste Alkyl und Alkenyl gegebenenfalls substituiert sind durch eine Gruppe der Formel $-NR^{11}R^{12}$,
worin
$R^{11}$ und $R^{12}$ gleich oder verschieden sind und Wasserstoff, Alkyl mit bis zu 4 Kohlenstoffatomen, Benzyl, Phenyl oder Acetyl bedeuten,
oder $R^8$
- für Phenyl steht, das bis zu 2-fach gleich oder verschieden substituiert sein kann durch Nitro, Fluor, Chlor, Methyl, Methoxy, Trifluormethyl, Trifluormethoxy, Amino, Alkylamino mit bis zu 2 Kohlenstoffatomen oder Dialkylamino mit jeweils bis zu 2 Kohlenstoffatomen je Alkylgruppe steht
und
A für einen geradkettigen, verzeigten oder cyclischen, gesättigten oder ungesättigten Kohlenwasserstoffrest mit bis zu 8 Kohlenstoffatomen steht, der gegebenenfalls durch ein Sauerstoff-oder Schwefelatom oder eine Gruppe $N-R^{14}$ unterbrochen ist, in der
$R^{14}$ Wasserstoff, Alkyl mit bis zu 2 Kohlenstoffatomen, Benzyl oder Phenethyl bedeuten kann,
und/oder substituiert sein kann durch Hydroxy, Carboxy, Alkoxycarbonyl mit bis zu 4 Kohlenstoffatomen oder durch Phenyl, das gegebenenfalls substituiert sein kann durch Halogen, Methyl, Methoxy, Trifluormethyl oder Trifluormethoxy
sowie deren physiologisch unbedenklichen Salze.

4. Verbindungen der allgemeinen Formel I gemäß Anspruch 1, in welcher

$R^1$ und $R^9$ jeweils für Methyl stehen
$R^2$ und $R^3$ gleich oder verschieden sind und für geradkettiges oder verzweigtes Alkyl mit bis zu 3 Kohlenstoffatomen stehen, das gegebenenfalls durch Phenyl substituiert ist, das seinerseits durch Methyl substituiert sein kann,
oder
$R^2$ und $R^3$ gemeinsam für Morpholino stehen
$R^4$ und $R^5$ jeweils für Wasserstoff stheen
$R^6$ für eine Gruppe der Formel
$-O-(CH_2)_n-R^{10}$, $-S-(CH_2)_n-R^{10}$, $-O-SO_2-(CH_2)_n-R^{10}$ oder $-O-CO-(CH_2)_n-R^{10}$
worin
n die Zahl 0, 1 oder 2 bedeutet und
$R^{10}$ Cyclohexyl oder Phenyl bedeutet, das gegebenenfalls durch Methyl substituiert ist
$R^7$ für Wasserstoff oder Alkyl mit bis zu 4 C-Atomen sthet
$R^8$ für Wasserstoff, Cyclopropyl, Cyclopentyl oder für geradkettiges oder verzweigtes Alkyl mit bis zu 5 Kohlenstoffatomen steht, das gegebenenfalls durch Hydroxy substituiert ist, und
A für die $CH_2$-$CH_2$-Gruppe steht.

5. Verfahren zur Herstellung von Verbindungen der allgemeinen Formel (I),

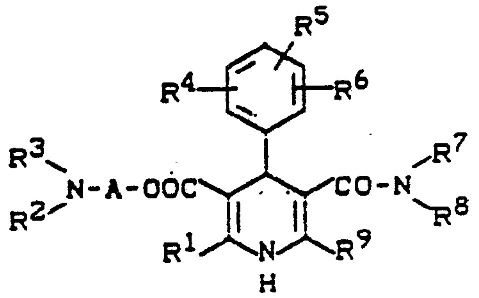
(I)

in welcher
$R^1$ und $R^9$ gleich oder verschieden sind und
- für geradkettiges, verzweigtes oder cyclisches Alkyl mit bis zu 6 Kohlenstoffatomen stehen, das gegebenenfalls durch Hydroxy, Cyano, Phenyl oder Halogen substituiert ist, oder
- für Cyano oder Phenyl stehen,
$R^2$ und $R^3$ gleich oder verschieden sind und für Wasserstoff, für geradkettiges oder verzweigtes Alkyl, Alkenyl oder Alkinyl mit jeweils bis zu 12 Kohlenstoffatomen stehen, das substituiert sein kann durch Halogen, Hydroxy, Alkoxy mit bis zu 8 Kohlenstoffatomen, Cyano, Trifluormethyl, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, durch Phenyl, welches gegebenenfalls durch Nitro, Phenyl, Cyano, Trifluormethyl, Trifluormethoxy, Alkyl mit bis zu 4 Kohlenstoffatomen, Halogen oder Alkoxy mit bis zu 4 Kohlenstoffatomen substituiert ist, für Cycloalkyl mit 3 bis 8 Kohlenstoffatomen oder
- für Aryl mit 6 bis 12 Kohlenstoffatomen stehendas bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Carbamoyl, Dialkylcarbamoyl mit jeweils bis zu 6 Kohlenstoffatomen je Alkylgruppe, Trifluormethyl, Trifluormethoxy, Difluormethoxy, Trifluormethylthio, Amino, Alkylamino mit bis zu 8 Kohlenstoffatomen, Dialkylamino mit jeweils bis zu 8 Kohlenstoffatomen je Alkylgruppe, Acetylamino oder Benzoylamino substituiert sein kann, oder $R^2$ und $R^3$ zusammen einen 5- bis 7-gliedrigen gesättigten oder ungesättigten heterocyclischen Ring bilden, der als Heteroatom ein Sauerstoffatom, ein Schwefelatom oder ein zusätzliches Stickstoffatom enthalten kann welches gegebenenfalls durch eine Gruppe $R^{13}$ substituiert sein kann, die für Wasserstoff, eine geradkettige oder verzweigte, gesättigte oder ungesättigte Alkylgruppe mit bis zu 10 Kohlenstoffatomen steht, die gegebenenfalls durch Phenyl, welches durch Halogen, Alkyl mit bis zu 4 C-Atomen, Alkoxy mit bis zu 4 C-Atomen, Nitro und Halogenalkyl mit bis zu 2 C-Atomen substituiert sein kann oder für Phenyl steht, das gegebenenfalls durch Halogen, Cyano, Nitro, Alkyl mit bis zu 2 C-Atomen, Alkoxy mit bis zu 2 C-Atomen oder Halogenalkyl mit bis zu 2 C-Atomen substituiert sein kann,
$R^4$ und $R^5$ gleich oder verschieden sind und
- für Wasserstoff, Halogen, Alkyl mit bis zu 6 Kohlenstoffatomen, Alkoxy mit bis zu 6 Kohlenstoffatomen,

Alkylthio mit bis zu 4 Kohlenstoffatomen, Cyano, Nitro, Dialkylamino mit jeweils bis zu 4 Kohlenstoffatomen je Alkylgruppe, Trifluormethyl, Trifluormethoxy, Difluormethoxy oder Trifluormethylthio stehen,
$R^6$ - für eine Gruppe der Formel $-O-(CH_2)n-R^{10}$, $-S-(CH_2)_n-R^{10}$, $-O-SO_2-(CH_2)_n-R^{10}$, $-O-CO-(CH_2)_n-R^{10}$ steht, worin
n - 0 bis 4 bedeutet,
und
$R^{10}$- Cyclohexyl bedeutet oder Aryl mit 6 bis 10 Kohlenstoffatomen bedeutet, das bis zu 4-fach gleich oder verschieden durch Halogen, Cyano, Nitro, Trifluormethyl, Trifluormethoxy, Difluormethoxy, Trifluormethylthio, Alkyl mit bis zu 6 Kohlenstoffatomen, Alkoxy mit bis zu 6 Kohlenstoffatomen, Alkylthio mit bis zu 4 Kohlenstoffatomen, Amino, Alkylamino mit bis zu 6 Kohlenstoffatomen, Dialkylamino mit jeweils bis zu 6 Kohlenstoffatomen je Alkylgruppe oder Acetylamino substituiert sein kann oder
- einen 5- bis 7-gliedrigen gesättigten oder ungesättigten heterocyclischen Ring bedeutet, der als Heteroatome ein Sauerstoffatom, ein Schwefelatom oder zwei Stickstoffatome enthalten kann,
und
$R^7$ und $R^8$ gleich oder verschieden sind und jeweils
- für Wasserstoff,
- für Cycloalkyl mit 3 bis 8 Kohlenstoffatomen oder
- für geradkettiges oder verzweigtes Alkyl oder Alkenyl mit jeweils bis zu 18 Kohlenstoffatomen stehen, die gegebenenfalls substituiert sind durch Halogen, Hydroxy, Alkoxy mit bis zu 8 Kohlenstoffatomen, Alkylthio mit bis zu 8 Kohlenstoffatomen, Alkylcarbonyl mit bis zu 8 Kohlenstoffatomen im Alkylrest, Carboxy oder Alkoxycarbonyl mit bis zu 8 Kohlenstoffatomen, Cyano oder durch Phenyl- oder Phenoxygruppen, welche gegebenenfalls durch Nitro, Cyano, Trifluormethyl, Trifluormethoxy, Alkyl mit bis zu 4 Kohlenstoffatomen oder Alkoxy mit bis zu 4 Kohlenstoffatomen substituiert sind, oder wobei die Reste Cycloalkyl, Alkyl oder Alkenyl gegebenenfalls durch eine Gruppe der Formel $-NR^{11}R^{12}$ substituiert sind,
worin
$R^{11}$ und $R^{12}$ gleich oder verschieden sind, und jeweils Wasserstoff, Alkyl mit bis zu 8 Kohlenstoffatomen, Aralkyl mit 7 bis 14 Kohlenstoffatomen, Aryl mit 6 bis 10 Kohlenstoffatomen bedeuten,
oder $R^7$ und $R^8$ jeweils
- für Aryl mit 6 bis 12 Kohlenstoffatomen stehen, das bis zu 3-fach gleich oder verschieden durch Nitro, Cyano, Halogen, Alkyl mit bis zu 6 Kohlenstoffatomen, Alkoxy mit bis zu 6 Kohlenstoffatomen, Alkylthio mit bis zu 6 Kohlenstoffatomen, Trifluormethyl, Trifluormethoxy, Difluormethoxy, Trifluormethylthio, Amino, oder
- für einen 5- bis 7-gliedrigen gesättigten oder ungesättigten heterocyclischen Ring stehen, der als zusätzliches Heteroatom ein Sauerstoffatom, ein Schwefelatom oder eine NH- oder N-Alkyl-Gruppe (1-4 C-Atome) enthalten kann, und
A für geradkettigen, verzweigten oder cyclischen, gesättigten oder ungesättigten Kohlenwasserstoffrest mit bis zu 12 Kohlenstoffatomen steht, der gegebenenfalls durch ein Sauerstoff- oder Schwefelatom oder eine Gruppe $N-R^{14}$ unterbrochen sein kann, in der
$R^{14}$ Wasserstoff, Alkyl bis zu 4 Kohlenstoffatomen, Benzyl oder Phenethyl bedeuten kann,
und/oder
der substituiert sein kann durch Halogen, Hydroxy, Acetoxy, Carboxy, Alkoxycarbonyl mit bis zu 8 Kohlenstoffatomen oder Phenyl, das gegebenenfalls substituiert sein kann durch Halogen, Alkyl mit bis zu 2 Kohlenstoffatomen, Alkoxy mit bis zu 2 Kohlenstoffatomen, Halogenmethyl, Halogenmethoxy, Hydroxy oder Cyano,
dadurch gekennzeichnet, daß man
[A] Aldehyde der allgemeinen Formel (II)

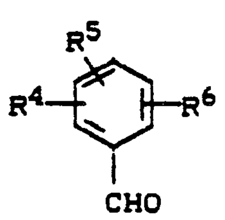

(II)

in welcher
$R^4$, $R^5$ und $R^6$ die oben angegebene Bedeutung haben
mit
$\beta$-Ketocarbonsäureestern der allgemeinen Formel (III)

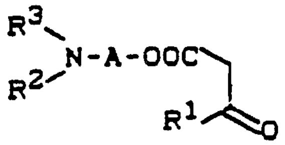

(III)

in welcher
$R^1$, $R^2$, $R^3$ und A die oben angegebene Bedeutung haben
gegebenenfalls nach Isolierung der hieraus entstehenden Ylidenverbindungen der allgemeinen Formel (IV)

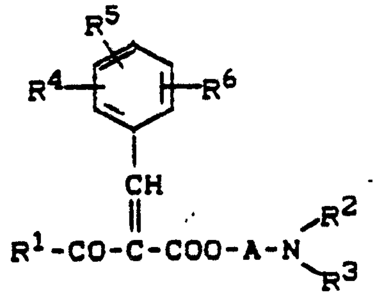

(IV)

in welcher
$R^1$, $R^2$, $R^3$, $R^4$, $R^5$, $R^6$ und A die oben angegebene Bedeutung haben,
mit
β-Ketocarbonsäureamiden der allgemeinen Formel (V)

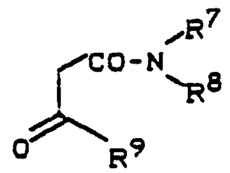

(V)

in welcher
$R^7$, $R^8$ und $R^9$ die oben angegebene Bedeutung haben
und Ammoniak bzw. direkt mit den hieraus hergestellten β-Aminocrotonsäureamiden der allgemeinen Formel (VI)

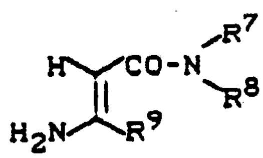

(VI)

in welcher
$R^7$, $R^8$ und $R^9$ die oben angegebene Bedeutung haben,
umsetzt,
oder indem man
[B] Aldehyde der allgemeinen Formel (II) und β-Ketocarbonsäureamiden der allgemeinen Formel (V) bzw. deren Ylidenverbindungen der allgemeinen Formel (VII)

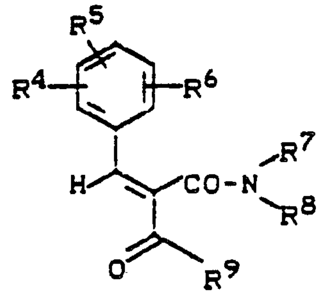

(VII)

in welcher
$R^4$, $R^5$, $R^6$, $R^7$, $R^8$ und $R^9$ die oben angegebene Bedeutung haben,
mit
β-Ketocarbonsäureestern der allgemeinen Formel (III) und Ammoniak bzw. direkt mit den hieraus hergestellten Aminocrotonsäureestern der allgemeinen Formel (VIII)

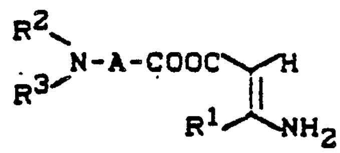

(VIII)

in welcher
$R^1$, $R^2$, $R^3$ und A die oben angegebene Bedeutung haben,
umsetzt
oder indem man
[C] Dihydropyridinmonocarbonsäuren der allgemeinen Formel (IXa) oder (IXb)

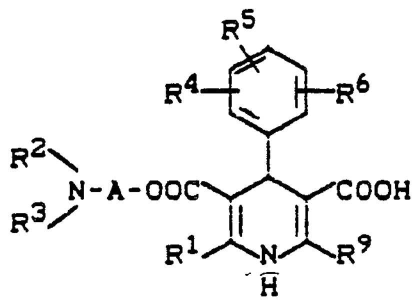

oder

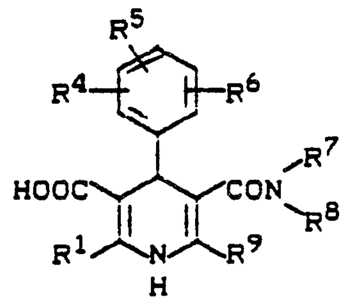

(IXa) (IXb)

in welchen
$R^1$, $R^2$, $R^3$, $R^4$, $R^5$, $R^6$, $R^7$, $R^8$, $R^9$ und A die oben angegebene Bedeutung haben,
gegebenenfalls über reaktive Säurederivate mit Aminen der allgemeinen Formel (Xa)

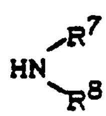

(Xa)

in welcher
$R^7$ und $R^8$ die oben angegebene Bedeutung haben,
bzw. mit Verbindungen der Formel (Xb)

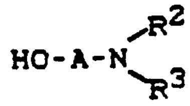

(Xb)

in welcher
$R^2$, $R^3$ und A die oben angegebene Bedeutung haben,
umsetzt, wobei (selbstverständlich) jeweils nur (IXa) mit (Xa) und (IXb) mit (Xb) umgesetzt wird.

6. Verfahren zur Herstellung von enantiomerenreinen Verbindungen der allgemeinen Formel (I), dadurch gekennzeichnet, daß man Diastereomerengemische der Formeln (XIa*) bzw. (XIb*)

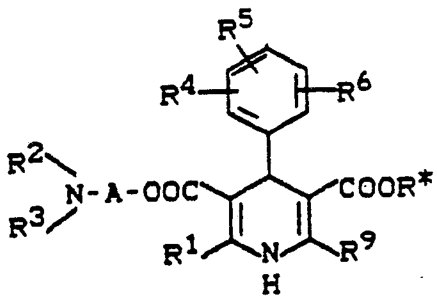

und

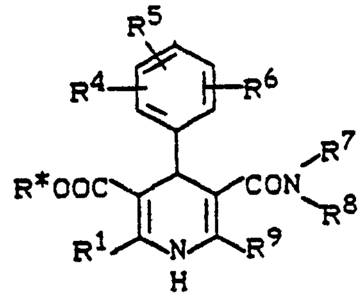

(XIa*) (XIb*)

in welcher
$R^1$, $R^2$, $R^3$, $R^4$, $R^5$, $R^6$, $R^7$, $R^8$, $R^9$ und A die oben angegebene Bedeutung haben und
R* einen optisch aktiven Esterrest darstellt, durch Kristallisation, Chromatographie oder Craig-Verteilung in die einzelnen Diastereomeren trennt,
gegebenenfalls den optisch aktiven Esterrest abspaltet und anschließend die enantiomerenreinen Carbonsäuren der Formeln (IXa*) und (IXb*)

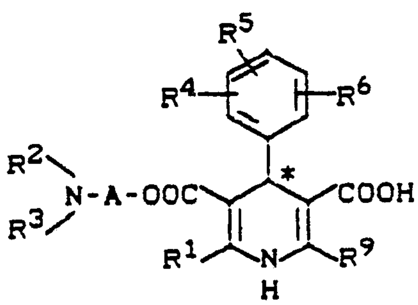

oder

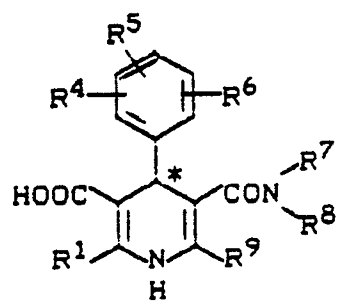

(IXa*) (IXb*)

herstellt, und die Verbindungen der allgemeinen Formel (IXa*) mit Verbindungen der allgemeinen Formel (Xa)

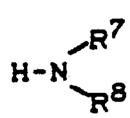

(Xa)

in welcher
$R^7$ und $R^8$ die oben angegebene Bedeutung haben
gegebenenfalls über aktivierte Säurederivate umsetzt,

oder indem man
Verbindungen der allgemeinen Formel (IXb') mit Verbindungen der allgemeinen Formel (Xb)

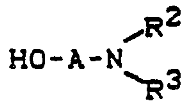

(Xb)

in welcher
$R^2$, $R^3$ und A die oben angegebene Bedeutung haben
gegebenenfalls über aktivierte Säurederivate umsetzt.

7. Basische 4-Aryl-DHP-amide der allgemeinen Formel (I) gemäß Anspruch 1 zur Verwendung bei der Bekämpfung von Kreislauferkrankungen.

8. Arzneimittel enthaltend mindestens eine Verbindung der allgemeinen Formel (I) gemäß Anspruch 1.

9. Verfahren zur Herstellung von Arzneimitteln, dadurch gekennzeichnet, daß man mindestens eine Verbindung der allgemeinen Formel (I) gemäß Anspruch 1 ebenfalls unter Verwendung von üblichen Hilfs- und Trägerstoffen in eine geeignete Applikationsform überführt.

10. Verwendung von Verbindungen der allgemeinen Formel (I) gemäß Anspruch 1 bei der Herstellung von Arzneimitteln zur Behandlung von Kreislauferkrankungen, Herzinsuffizienz, Diabetes und Ödemen.

11. Verwendung von Verbindungen der allgemeinen Formel (I) gemäß Anspruch 1 bei der Herstellung von Arzneimitteln zur Bekämpfung von Hypertonie, Herzrhythmusstörungen, Niereninsuffizienz, Leberzirrhose, Aszites, Ödemen, Glaukom und Diabetes.